# EUROPEAN PATENT SPECIFICATION

(11) **EP 4 073 513 B1**
(45) Date of publication and mention of the grant of the patent: **06.05.2026**
(21) Application number: 20845774.7
(22) Date of filing: 11.12.2020
(51) Int. Cl.: G01N 33/543, G01N 33/564, G01N 33/68, G01N 33/569

(54) **INFECTION ANALYSIS APPARATUS AND METHOD**
INFEKTIONSANALYSEVORRICHTUNG UND -VERFAHREN
APPAREIL ET PROCÉDÉ D'ANALYSE D'INFECTION

(30) Priority: 12.12.2019 GB 201918347
(43) Date of publication of application: 19.10.2022
(73) Proprietor: Aksense Ltd, London, EC2A 4NE (GB)
(72) Inventor: KARAKULLUKÇU, Asiye, Esenyurt Ístanbul (TR)
(74) Representative: Keltie LLP
(86) International application number: PCT/EP2020/085859
(87) International publication number: WO 2021/116465

(56) References cited:
- EP-A1- 2 997 371
- WO-A2-2006/109311
- US-A1- 2017 108 493
- "Molecular Biology of the Cell", 1 January 2015, GARLAND SCIENCE, ISBN: 978-0-8153-4432-2, article ALBERTS BRUCE ET AL: "Chapter 1: Cells and Genomes & Chapter 2: Cell Chemistry and Bioenergetics", pages: 1 - 107, XP093259801
- KIM SANG WOO ET AL: "Serum resistance of Acinetobacter baumannii through the binding of factor H to outer membrane proteins", FEMS MICROBIOLOGY LETTERS, vol. 301, no. 2, 1 December 2009 (2009-12-01), pages 224 - 231, XP093259817, ISSN: 0378-1097, DOI: 10.1111/j.1574-6968.2009.01820.x
- GUPTA BHUPENDER S. ET AL: "Buffers more than buffering agent: introducing a new class of stabilizers for the protein BSA", PHYSICAL CHEMISTRY CHEMICAL PHYSICS, vol. 17, no. 2, 1 January 2015 (2015-01-01), pages 1114 - 1133, XP093259818, ISSN: 1463-9076, DOI: 10.1039/C4CP04663C
- ISLAM A H M S ET AL: "Demonstration of an outer membrane protein that is antigenically specific for Acinetobacter baumannii", DIAGNOSTIC MICROBIOLOGY AND INFECTIOUS DISEASE, ELSEVIER, AMSTERDAM, NL, vol. 69, no. 1, 1 January 2011 (2011-01-01), pages 38 - 44, XP027554288, ISSN: 0732-8893, [retrieved on 20101209]
- CROSAT JORGE H ET AL: "Outer Membrane Proteins Induced Under Conditions of Iron Limnitation in the Marine Fish Pathogen Vibrio anguillarum 775", INFECTION AND IMMUNITY, vol. 31, no. 1, 1 January 1981 (1981-01-01), pages 223 - 227, XP093259820
- ALBERTI SEBASTIAN ET AL: "Analysis of Complement C3 Deposition and Degradation on Klebsiella pneumoniae", INFECTION AND IMMUNITY, vol. 64, no. 11, 1 November 1996 (1996-11-01), pages 4726 - 4732, XP093259821
- GULABOSKI RUBIN ET AL: "Protein film voltammetry: electrochemical enzymatic spectroscopy. A review on recent progress", JOURNAL OF SOLID STATE ELECTROCHEMISTRY, vol. 16, no. 7, 4 May 2011 (2011-05-04), DE, pages 2315 - 2328, XP093259929, ISSN: 1432-8488, Retrieved from the Internet <URL:http://link.springer.com/article/10.1007/s10008-011-1397-5/fulltext.html> DOI: 10.1007/s10008-011-1397-5
- NICHOLAS D J YATES ET AL: "Methodologies for "Wiring" Redox Proteins/Enzymes to Electrode Surfaces", CHEMISTRY - A EUROPEAN JOURNAL, JOHN WILEY & SONS, INC, DE, vol. 24, no. 47, 6 June 2018 (2018-06-06), pages 12164 - 12182, XP071847324, ISSN: 0947-6539, DOI: 10.1002/CHEM.201800750
- LEI CUN-XI ET AL: "Amperometric immunosensor for probing complement III (C3) based on immobilizing C3 antibody to a nano-Au monolayer supported by sol-gel-derived carbon ceramic electrode", ANALYTICA CHIMICA ACTA, vol. 513, no. 2, 1 June 2004 (2004-06-01), AMSTERDAM, NL, pages 379 - 384, XP055789117, ISSN: 0003-2670, DOI: 10.1016/j.aca.2004.01.029
- KATZ E ET AL: "PROBING BIOMOLECULAR INTERACTIONS AT CONDUCTIVE AND SEMICONDUCTIVE SURFACES BY IMPEDANCE SPECTROSCOPY: ROUTES TO IMPEDIMETRIC IMMUNOSENSORS, DNA-SENSORS, AND ENZYME BIOSENSORS", ELECTROANALYSIS, VHC PUBLISHERS, INC, US, vol. 15, no. 11, 1 January 2003 (2003-01-01), pages 913 - 947, XP009021944, ISSN: 1040-0397, DOI: 10.1002/ELAN.200390114

## Description

### Field of the invention

The present invention relates to the field of infection analysis. In particular, embodiments of the invention relate to analysis of a biological sample, for example a blood sample, from a person to determine whether an infection, such as a bacterial infection, is present or not in the body of the person.

### Background

Infections are caused by foreign agents in a body of an organism, for example a human person or patient, and can result in illness or disease of the organism. Pathogens, e.g. pathogenic bacteria provide one of the ways in which infections can be spread. Infections can be characterised by the pathogen that cause them, which may be a specific pathogenic strain, for example a bacterial strain. Evidence of the infection can be present in the blood, urine, or other parts of the human body.

Detecting such infections in an organism, for example a human patient, from a biological sample, (e.g. a blood sample or urine sample) can be useful for diagnosis and subsequent treatment of the infection to prevent or alleviate the illness or disease. Once it is known that an infection is present in the patient, treatments such as antibiotics can be used to cure the infection.

Commercial methods of determining whether a patient has an infection, e.g. a bacterial infection, entail first taking a sample of the patient's blood, urine, tissue, or body fluids from the suspected infected area. The sample is then cultured to grow the pathogen, e.g. bacteria, and subsequently identify the pathogen strain, e.g. pathogenic bacteria, in order to diagnose the infection.

This is a time consuming process, resulting in long waiting periods to determine whether a patient has an infection or not. The longer a patient is waiting without a determination, the more chance an infection has to spread. There is therefore a desire to obtain a quick determination of infection to stop it spreading and to cure a patient as soon as possible.

US 2017/108493 and WO 2006/109311 describe systems and methods for detecting infection of a pathogen.

### Summary

Aspects of the invention are set out in the independent claims and preferred features are set out in the dependent claims. Hereinafter aspects and embodiments are aspects and embodiments of the invention only if they fall within the scope of the claims.

According to a first aspect there is provided a system for detecting an infection with a bacterial pathogen strain, the system comprising: an electrode functionalised with the surface proteins of the bacterial pathogen strain and a controller configured to communicate with the electrode to perform an electrochemical test of a biological sample from a subject, the biological sample deposited on the electrode, wherein the electrochemical test measures a binding energy of one or more biomarkers in the biological sample with the surface proteins of the bacterial pathogen strain functionalised on the electrode to determine whether the subject has an immune response to the bacterial pathogen strain indicative of an infection with the bacterial pathogen strain wherein the controller is an electronic device, wherein the biomarkers comprise one or more of C3b, IgG1 and/or IgG3; and wherein the system is configured to determine whether the subject has an infection with the bacteria based on a determination that the binding energy exceeds a threshold value. Optionally, the subject is a patient. Optionally, the biological sample is a blood sample.

In some embodiments, there is provided a system for detecting an infection caused by a strain of bacteria, the system comprising: an electrode functionalised with proteins isolated from a cell wall of a bacteria of the bacterial strain; and a controller configured to communicate with the electrode to perform an electrochemical test of a blood sample from a subject, the blood sample deposited on the electrode, wherein the electrochemical test measures a binding energy of one or more biomarkers in the blood sample with the proteins functionalised on the electrode to determine whether the subject has or has had a response to the strain of bacteria indicative of an infection caused by the strain of bacteria. Optionally, the subject is a patient. Optionally the response is an immune response to the strain of bacteria indicative of an infection caused by the strain of bacteria.

In some embodiments, there is provided a system for detecting an infection caused by a strain of bacteria, the system comprising: an electrode functionalised with surface proteins isolated from a cell wall of a bacteria of the bacterial strain; and a controller configured to communicate with the electrode to perform an electrochemical test of a blood sample from a patient, the blood sample deposited on the electrode, wherein the electrochemical test measures a binding energy of one or more biomarkers, in the blood sample with the surface proteins functionalised on the electrode to determine whether the patient has or has had an immune response to the strain of bacteria indicative of an infection caused by the strain of bacteria.

Advantageously, the proteins isolated from the cell wall of the strain of bacteria, or the surface proteins of the pathogen strain, used to functionalise the electrode comprise proteins that specifically bind to the one or more biomarkers present or active in a patient's blood or other biological sample if the patient has been infected by the bacterial pathogen strain. The biomarker is present in the patient as an inactive precursor and is activated or stimulated if the patient has been infected by the pathogen strain, optionally irreversibly activated. The biomarkers IgG1, IgG3 and C3b are absent or inactive in the serum of non-infected people, whereas in the serum of patients with bacterial infections, they are directed against surface proteins of the bacteria.

The biomarker has an altered, optionally increased, binding energy to the proteins isolated from the cell wall, or to the surface proteins of the pathogen strain, if the patient has an immune response to the bacterial pathogen strain. If the patient has not been infected by the bacterial pathogen strain, there will be reduced binding or no binding of the biological sample (e.g. blood sample) with the surface proteins of the electrode. The degree to which specific biomarkers in the biological sample bind to the surface proteins of the pathogen strain is related to whether an infection of the bacterial pathogenic strain is present or absent.

This may be the result of the complement pathway, opsonisation, and/or macrophage activation. Opsonization of bacteria with the biomarkers IgG1 and IgG3 is accompanied by an increase of hydrophobicity of the bacterial surface. The biomarker C3b binds covalently to the pathogen surface. IgG1, IgG3 and C3b also bind in a specific receptor-ligand manner with complementary membrane receptors on the surface of phagocytes involved in phagocytosis, largely during the adhesive phase.

In this manner, measurement of a binding energy to surface proteins on the electrode, for example isolated from the cell wall of a strain of bacteria, upon deposition of a patient's biological sample (e.g. blood sample) onto the electrode, can be used to determine whether a patient has an infection of the bacterial pathogen strain (e.g. strain of bacteria). Absence of a binding energy or measurement of an energy under the discovered threshold of the binding energy between the functionalised electrode, optionally surface proteins on the electrode, and the biological sample (e.g. blood sample) is correspondingly indicative that a patient is not infected by the bacterial pathogen (e.g. bacteria) that is being tested for. Specific counter-ligands are bound with a high energy, implying an intimate stereochemical relationship and the binding energy exceeds a threshold value.

Quick and accurate results can be achieved using the described bacterial infection detecting system. The binding detected between the electrode and the biological sample (e.g. blood sample) deposited thereon provides effective means of determining a true result. As such, resulting diagnosis can be generated with a high degree of accuracy. In addition, the binding energy is measured quantitatively and may indicate the infection level in addition to detecting the infection. The time taken to get a result can be as quick as a few seconds, meaning that infections can be quickly diagnosed and treated. This prevents or alleviates prolonged illness of the patient and in turn aids the prevention of the infection spreading to others, particularly those in the vicinity of or that make contact with infected patient.

The proteins used to functionalise the electrode may be isolated from the bacterial pathogen strain or may be produced in a recombinant manner.

It will be further understood that the sample may be any suitable sample, for example a urine sample, or endotracheal aspiration sample, bronchoalveolar lavage sample or other extracellular fluid sample of the person, instead of a blood sample.

Electrochemical testing may comprise application of a current or voltage to the functionalised electrode comprising the deposited biological sample (e.g. blood sample). The controller can be configured to apply the conditions of the electrochemical testing to the electrode.

The biomarker, for example present in the patient's blood, comprises proteins, , specifically C3b, IgG1 and/or IgG3 proteins.

In some embodiments, the biomarkers comprise one or more opsonins, optionally wherein the system is configured to measure the binding energy of the surface proteins functionalised on the electrode with the one or more opsonins in the biological sample (e.g. blood sample). An "opsonin" as referred to herein is any molecule that is involved in opsonization and/or binds to a pathogen cell and promotes phagocytosis. Examples of opsonins are C3b, and Immunoglobulin G, IgG1 and IgG3, proteins.

The proteins comprise C3b and/or IgG1 and IgG3. C3b proteins bind pathogen surface proteins with a strong and specific binding energy (high affinity), and then CR1 binds to C3b promoting phagocytosis of the opsonized bacterial cell. IgG, such as IgG1 and IgG3, bind bacterial surface proteins (e.g. cell-wall proteins) via their Fab or Fc region, with a strong and specific binding energy (high affinity). FcyRI binds to IgG1 and IgG3, promoting phagocytosis of the opsonized pathogen (e.g. bacterial cell). Anti-human FcyRI can be used to recognise binding, e.g. of IgG1 and IgG3, to the surface of the bacterial pathogen (e.g. to the bacterial cell wall) for opsonisation.

Wherein the biomarker is a protein comprising one or more C3b and/or IgG1 or 3, proteins, the system can be configured to measure the binding energy of the isolated proteins functionalised on the electrode with C3b and/or I IgG1 or 3 in the blood sample.

The biomarker comprises C3b, IgG1 and/or IgG3, wherein the system is configured to measure the binding energy of the surface proteins functionalised on the electrode with C3b, IgG1 and/or IgG3 in the biological sample (e.g. blood sample).

In some embodiments, just the C3b proteins in the blood sample may bind with the proteins on the functionalised surface. In other examples, IgG1 and IgG3 proteins may bind to the proteins on the functionalised surface. In some embodiments, C3b and IgG1 and IgG3, proteins bind to the proteins on the functionalised surface.

In some embodiments, the biomarker comprises one or more complement proteins, optionally complement proteins which are present in the blood of non-infected patients as inactive precursors but are rapidly activated upon contact with a pathogen, e.g. bacterial cells, when the patient is infected. In some embodiments, the biomarker comprises one or more complement proteins, such as C3b. In some embodiments, the biomarker comprises C3b, optionally wherein the system is configured to measure the binding energy of the proteins functionalised on the electrode with C3b in the biological sample (e.g. blood sample).

In other embodiments, the biomarker optionally comprises antibodies, such as IgG1 and IgG3, and the system can be configured to measure the binding energy of the proteins functionalised on the electrode with the antibodies in the blood sample.

In some embodiments, the proteins isolated from the bacterial cell wall or the surface proteins of the pathogen strain, for example the proteins that bind the biomarker, comprise one or more of: complement binding proteins (e.g. C3b binding proteins), immunoglobulin (e.g. IgG1 and/or IgG3) binding proteins, CR1 binding proteins, FcR (e.g. FcyRI) binding proteins, OprF, CipA, TSR domains, Extracellular complement-binding proteins, LPS-binding proteins, 0-N-acetyl-D-glucosamine, Protein A, Immunoglobulin-binding protein 1 or surface proteins, membrane proteins, outer membrane proteins (e.g. outer membrane protein A), teichoic acid, 0 somatic antigens, receptor binding proteins. Optionally, wherein the isolated protein may be a receptor of the biomarker, wherein the biomarker is preferably a C3b and/or IgG protein. i.e, wherein the biomarker is C3b, IgG1 and/or IgG3.

In preferred embodiments, the surface proteins or proteins isolated from the bacterial cell wall, comprise one or more of: complement binding proteins (e.g. C3b binding proteins), immunoglobulin (IgG1 and/or IgG3) binding proteins, CR1 binding proteins, and FcR (e.g. FcyRI) binding proteins.

In some embodiments, the surface proteins or proteins isolated from the bacterial cell wall, comprise C3b binding proteins and optionally CR1 binding proteins. In some embodiments, the surface proteins or proteins isolated from the bacterial cell wall, comprise IgG1/ IgG3 binding proteins and optionally FcyRI binding proteins.

While not encompassed by the claims of the present disclosure, the surface proteins of a virus may be used to functionalise the electrode. These surface proteins may comprise one or more of: spike (S) proteins, envelope proteins, capsid proteins, glycoproteins, receptor binding proteins, optionally wherein the proteins are recombinant proteins.

The system is optionally configured to determine whether the patient has a bacterial infection based on a determination that the binding energy exceeds a threshold value. The threshold value can be given as a voltammetric or amperometric value or as a percentage. For example, wherein the threshold value has been previously determined for the bacterial pathogen in order to achieve the desired accuracy for detecting an infection. The thresholds may be calculated based on infected and non- infected subjects and corresponding samples taken therefrom. Providing a threshold value, in particular a threshold value that has been specifically determined for an individual bacterial pathogen strain, can therefore be advantageous in providing a tailored system. A more representative result can be achieved using this tailored system, leading to a more accurate determination of infection.

The controller may be configured to select the threshold value based on the bacterial infection being detected. For example, an infection to be detected may be selectable from a list of infections via a user interface of the controller. The user interface may be a screen, for example a touch screen, and may be in communication with the controller over a wireless or wired connection. The user interface may, for example, be presented on an electronic device such as a computer or mobile phone.

The threshold value may be between -3.4 V and 3.4 V. The threshold value may optionally be unique to each bacterial strain.

The strain of bacteria can be selected from a list of: *Klebsiella pneumonia; Escherichia coli; Pseudomonas aeruginosa; Acinetobacter baumannii; Enterococcus faecalis; Enterococcus faecium;* and *Staphylococcus aureus.* It will be appreciated that other bacterial strains can be tested for using the above system. Threshold values of other bacterial strains may be different to those above and may extend beyond the range of - 3.4 V to 3.4 V in either direction.

Optionally, the proteins isolated from the bacterial cell wall, or the surface proteins of the pathogen strain, are immobilized on a surface of the electrode. Immobilization of the proteins, for example isolated from the bacterial cell wall, comprises chemically binding the proteins to the electrode in fixed locations of the surface, wherein the surface is the surface of the electrode onto which the biological sample (e.g. blood sample) is to be deposited for testing. The immobilized proteins can be located at a localised area of the electrode, for example a working area of the electrode. Preferably the proteins are immobilized at a fixed concentration on the electrode.

The immobilized proteins, for example isolated from the bacterial cell wall, may comprise a coating on the surface of the electrode. The coating may be manufactured on the surface using known techniques.

The electrode optionally comprises a plurality of conductive elements and wherein the controller is configured to perform the electrochemical test by measuring the binding energy of biomarkers, for example proteins, in the biological sample (e.g. blood sample) with the proteins functionalised on the electrode by applying a voltage differential across at least some of the plurality of conductive elements. Optionally, wherein the conductive elements are functionalised with the isolated proteins of the cell wall extracted from the bacteria, or with the surface proteins of the bacterial pathogen strain.

Preferably, the electrode is a carbon electrode, optionally modified with gold nanoparticles to enhance the electron transfer rate and provide a greater surface area such that a higher number of proteins can be immobilized to improve the response signal.

According to a second aspect, there is provided a method for detecting an infection with a bacterial pathogenic strain, the method comprising: functionalising an electrode with surface proteins of the bacterial pathogenic strain, and performing an electrochemical test of a biological sample from a subject, the biological sample deposited on the electrode, wherein the electrochemical test comprises measuring a binding energy of one or more biomarkers in the biological sample with the surface proteins functionalised on the electrode to determine whether the subject has an immune response indicative of an infection with said bacterial pathogen strain, wherein the biomarkers comprise one or more of C3b, IgG1 and/or IgG3; and wherein the method comprises determining whether the subject has an infection with the bacteria based on a determination that the binding energy exceeds a threshold value.

In some embodiments, there is provided a method of detecting an infection caused by a strain of bacteria, the method comprising: functionalising an electrode with proteins isolated from a cell wall of a bacteria of the strain of bacteria; and performing an electrochemical test of a blood sample from a subject, the blood sample deposited on the electrode, wherein the electrochemical test comprises measuring a binding energy of one or more biomarkers in the blood sample with the proteins functionalised on the electrode to determine whether the subject has or has had a response to the strain of bacteria indicative of an infection caused by the strain of bacteria. Optionally, wherein the subject is a patient.

In some embodiments, there is provided a method of detecting an infection caused by a strain of bacteria, the method comprising: functionalising an electrode with proteins isolated from a cell wall of a bacteria of the strain of bacteria; and performing an electrochemical test of a blood sample from a patient, the blood sample deposited on the electrode, wherein the electrochemical test comprises measuring a binding energy of one or more biomarkers in the blood sample with the proteins functionalised on the electrode to determine whether the patient has or has had an immune response to the strain of bacteria indicative of an infection caused by the strain of bacteria.

While not encompassed by the claims of the present disclosure, there is provided a method for detecting an infection caused by a virus, the method comprising: functionalising an electrode with recombinant surface proteins of the virus and performing an electrochemical test of a biological sample from a patient, the biological sample deposited on the electrode, wherein the electrochemical test comprises measuring a binding energy of one or more biomarkers in the biological sample with the proteins functionalised on the electrode to determine whether the patient has an infection caused by said virus.

The proteins used to functionalise the electrode may be isolated from the bacterial pathogen strain or may be produced in a recombinant manner.

The biomarker, for example present in the patient's blood, comprises proteins, specifically the biomarkers comprise C3b, IgG1 and/or IgG3.

Preferably, wherein the proteins comprise C3b, IgG1 and/or IgG3. The Fc-gamma receptor 1 binds the Fc region of Immunoglobulin G (IgG) which binds the bacterial cell wall.

Optionally, wherein the biomarker is a protein, the protein preferably comprising one or more C3b, and/or IgG proteins, measuring the binding energy of the proteins functionalised on the electrode with C3b, and/or IgG proteins in the blood sample. In some embodiments, just the C3b proteins in the blood sample may bind with the proteins on the functionalised surface. In other examples, just the IgG proteins may bind to the proteins on the functionalised surface. In some embodiments, both C3b and IgG proteins bind to the proteins on the functionalised surface. It will be appreciated that other protein binding combinations may be possible, for example with different complement proteins such as C5a, C4b, iC3b, C1q, C5a, CR1, C1q, C1s, C1r, 2 proteinases, IgG1, IgG3 or Fc receptors such as Fc-gamma receptor 1, FcyRI.

In some embodiments, the biomarker comprises complement pathway proteins which are present in the blood of non-infected patients as inactive precursors. These complement proteins are rapidly activated upon contact with a pathogen, e.g. bacterial cells, when the patient is infected.

In other embodiments, the biomarker optionally comprises antibodies. The method may further comprise measuring a binding energy of the proteins functionalised on the electrode with antibodies in the blood sample.

In some embodiments, the proteins isolated from the bacterial cell wall, or the surface proteins of the pathogen strain, for example the proteins that bind the biomarker, comprise one or more of: complement binding proteins (e.g. C3b binding proteins), immunoglobulin (e.g. IgG1 and/or IgG3) binding proteins, CR1 binding proteins, FcR (e.g. FcyRI) binding proteins, OprF, CipA, TSR domains, Extracellular complement-binding proteins, LPS-binding proteins, O-N-acetyl-D-glucosamine, Protein A, Immunoglobulin-binding protein 1 or surface proteins, membrane proteins, outer membrane proteins (e.g. outer membrane protein A), teichoic acid, 0 somatic antigens, receptor binding proteins. Optionally, wherein the isolated protein may be a receptor of the biomarker. Wherein the biomarker is C3b, IgG1 and/or IgG3. Optionally, wherein C3b binds to one of C3b binding proteins, outer membrane protein A, OprF, CipA, TSR domains, Extracellular complement-binding proteins, LPS-binding proteins or O-N-acetyl-D- glucosamine and Immunoglobulin G (IgG1 and/or IgG3) binds to immunoglobulin binding proteins, Protein A, Immunoglobulin-binding protein 1 or surface proteins.

In preferred embodiments, the surface proteins or proteins isolated from the bacterial cell wall, comprise one or more of: complement binding proteins (e.g. C3b binding proteins), immunoglobulin (IgG1 and/or IgG3) binding proteins, CR1 binding proteins, and FcR (e.g. FcyRI) binding proteins.

In some embodiments, the surface proteins or proteins isolated from the bacterial cell wall, comprise C3b binding proteins and optionally CR1 binding proteins. In some embodiments, the surface proteins or proteins isolated from the bacterial cell wall, comprise IgG1/ IgG3 binding proteins and optionally FcyRI binding proteins.

While not encompassed by the claims of the present disclosure, the surface proteins of the virus, used to functionalise the electrode, comprise one or more of: spike (S) proteins, envelope proteins, capsid proteins, glycoproteins, receptor binding proteins, optionally wherein the proteins are recombinant proteins.

In some embodiments the electrodes may be functionalised with recombinant protein parts, for example recombinant protein binding domains.

Optionally, determining whether the patient has a bacterial infection is based on a determination that the binding energy exceeds a threshold value. For example, wherein the threshold value has been previously determined for the bacterial pathogen in order to achieve the desired accuracy for detecting an infection. The controller may be configured to select the threshold value based on the infection being detected. Further optionally determining whether the patient has or has had an immune response to the bacterial pathogen based on a determination that the binding energy exceeds a threshold value. The threshold value may be selected based on the infection being detected. Wherein the threshold value can be between - 3.4 V and 3.4 V. The threshold value can be given as a voltammetric or amperometric value or as a percentage.

The strain of bacteria can be selected from a list of: *Klebsiella pneumonia; Escherichia coli; Pseudomonas aeruginosa; Acinetobacter baumannii; Enterococcus faecalis; Enterococcus faecium;* and *Staphylococcus aureus.* It will be appreciated that other bacterial strains can be tested for using the above system. Threshold values of other bacterial strains may be different to those above and may extend beyond the range of - 3.4 V to 3.4 V in either direction.

Functionalising the electrode may comprise immobilizing proteins isolated from the bacterial cell wall (or the surface proteins of the pathogen strain) onto a surface of the electrode. Optionally, wherein the surface is the surface of the electrode onto which the biological sample (e.g. blood sample) is deposited for testing. Functionalising the electrode optionally further comprises coating the surface of the electrode with the proteins isolated from the bacterial cell wall (or the surface proteins of the pathogen strain. Preferably the proteins are immobilized at a fixed concentration on the electrode.

The method may comprise a plurality of conductive elements and wherein the method may comprise performing an electrochemical test by measuring a binding energy of the biomarkers, for example proteins, in the biological sample (e.g. blood sample) with the proteins functionalised on the electrode by applying a voltage differential across at least some of the plurality of conductive elements. Optionally, wherein the conductive elements are functionalised with the proteins isolated from the bacterial cell wall, or with the surface proteins of the pathogen strain.

Preferably, the electrode is a carbon electrode, optionally modified with gold nanoparticles to enhance the electron transfer rate and provide a greater surface area such that a higher number of proteins can be immobilized to improve the response signal.

While not encompassed by the claims of the present disclosure, further described herein is a method to measure an infection level, the method comprising: functionalising an electrode with proteins of a pathogen strain, and performing an electrochemical test of a biological sample from a patient, the biological sample deposited on the electrode, wherein the electrochemical test comprises measuring a binding energy of one or more biomarkers in the biological sample with the proteins functionalised on the electrode to determine the level of infection caused by said pathogen strain in the patient.

The method may further comprise evaluating the response of a patient to a treatment/therapy based on the change in the level of infection as a result of the treatment/therapy. Optionally, a decrease in the binding energy of one or more biomarkers in the biological sample with the proteins functionalise on the electrode is indicative of a positive response to a treatment/therapy.

While not encompassed by the claims of the present disclosure, further described herein is a method of manufacturing an electrode for detecting an infection caused by a pathogen strain, the method comprising immobilizing proteins of the pathogen strain onto the electrode.

While not encompassed by the claims of the present disclosure, further described herein is a method of manufacturing an electrode for detecting an infection caused by a strain of bacteria, the method comprising immobilizing proteins isolated from a cell wall of a bacteria of the strain of bacteria onto the electrode. The cell walls of the bacteria of the functionalised electrode may correspond to the bacteria, and hence the infection, to be detected.

The method may further comprise extracting the surface proteins of the pathogen strain, or extracting the proteins isolated from a cell wall of a bacteria of the strain of bacteria, to be immobilized on the electrode, wherein extracting comprises one or more of:
culturing the pathogens until a log phase is reached;
harvesting the cells by centrifugation, optionally at their late log phase OD value;
disrupting the cells with glass beads, optionally 150-212 micron glass beads;
centrifugation to remove unbroken cells;
ultracentrifuge the supernatant to collect the proteins;
determine the molecular size of pathogens surface proteins by SDS-PAGE;
determine the C3b and IgG1/IgG3 specific binding proteins with Western Blot;
purifying the C3b and IgG1/IgG3 binding proteins with gel filtration column, ion exchange column, immunoprecipitation, and ultrafiltration, respectively;
re-apply the Western Blot process to determine which protein stays in the solution and the targeted one is there;
dialyze for removal of elution agents.

Optionally, the method may further comprise extracting the cell wall of the bacteria comprising the proteins to be immobilized on the electrode, wherein extracting comprises one or more of: incubating cells of the bacteria in a nutrient broth; collecting the cells by centrifugation; suspending the bacteria in a buffer; mixing the suspension; lysing the cells by vortexing of the mixed suspension; aspirating the cell lysate and removing unbroken cells from the mixture using centrifugation; obtaining cell envelopes by ultracentrifugation; adding HEPES (4-(2-hydroxyethyl)-1-piperazineethanesulfonic acid) to separate a cytoplasmic membrane from an outer membrane of the cell envelopes; and determining the presence and location of the protein.

Optionally, wherein the extracting as mentioned above comprises one or more of: incubating cells of the bacteria in a nutrient broth by growing the cells until a log phase is reached; collecting the bacterial cells by centrifugation; suspending the bacteria in a HEPES buffer; mixing the suspension with DNAse and RNAse; lysing the cells by vortexing using glass beads of the mixed suspension; aspirating the cell lysate and removing unbroken cells from the mixture using centrifugation; obtaining cell envelopes by ultracentrifugation; adding HEPES to separate a cytoplasmic membrane from an outer membrane of the cell envelopes; storing the inner cytoplasmic membrane at a temperature of -20 degrees Celsius; and determining the presence and location of the protein.

While not encompassed by the claims of the present disclosure, further described herein is a method of manufacturing an electrode for detecting an infection caused by a strain of virus, the method comprising immobilizing recombinant surface proteins of the virus onto the electrode. In some embodiments, the surface proteins of the virus, used to functionalise the electrode, comprise one or more of: spike (S) proteins, envelope proteins, capsid proteins, glycoproteins, receptor binding proteins, optionally wherein the proteins are recombinant proteins.

The method may further comprise obtaining the recombinant surface proteins of the virus by isolating the viral RNA of an infected cell, synthesising cDNA and expressing the viral cDNA in a host cell, optionally E. coli. Optionally, wherein the viral cDNA is expressed using a SUMO fusion system and Getaway E. coli expression system to produce the recombinant viral protein.

This method, which is not part of the invention, may further comprise identifying the proteins to be isolated from the cell wall of the bacteria (or the surface proteins of the pathogen strain) which specifically bind to one or more biomarkers, for example proteins e.g. C3b, IgG1, IgG3 , the method optionally comprising gel electrophoreses and/or Western Blotting.

Optionally, wherein the gel electrophoreses comprises one or more of the following: suspending the isolated proteins with a buffer containing sodium dodecyl sulfate-polyacrylamide, SDS-PAGE, and Coomassie blue; loading a strip of gel with the isolated proteins; and separating the proteins by gel electrophoreses.

Optionally, wherein Western Blotting comprises one or more of the following:electrophoretic transfer of the separated proteins to a membrane, optionally a nitrocellulose membrane or a PVDF membrane;
(a) cutting the membrane, e.g. nitrocellulose membrane, into strips
(b) incubating the strips with human serum dilutions comprising infection positive and infection negative serum;
(c) washing the strips and performing a second incubation in a solution comprising anti-human-C3b antibody, CR1 protein, anti-human-IgG1/3 antibody and/or FcR (e.g. FcyRI);
(d) washing the strips and performing a third incubation with an enzymeconjugated antibody, for example a Horseradish peroxidase (HRP) conjugated antibody
(e) washing the strips and performing a third incubation with a substrate solution, e.g. a Horseradish peroxidase, HRP, conjugated substrate; rinsing the strips with distilled water.
(f) visualization to determine the molecular weight of the proteins.

For Western Blotting to identify surface proteins which specifically bind to C3b, step (d) above comprises washing the strips and performing a second incubation in a solution comprising anti-human-C3b antibody and/or CR1 protein. In some embodiments, the method comprises two Western Blots, the first Western Blot comprising as step (d): washing the strips and performing a second incubation in a solution comprising anti-human-C3b antibody; and the second Western Blot comprising as step (d): washing the strips and performing a second incubation in a solution comprising CR1 protein, as a supportive and confirmative experiment.

For Western Blotting to identify surface proteins which specifically bind to IgG1/IgG3, step (d) above comprises washing the strips and performing a second incubation in a solution comprising anti-human-IgG1/3 antibody and/or FcR. In some embodiments, the method comprises two Western Blots, the first Western Blot comprising as step (d): washing the strips and performing a second incubation in a solution comprising anti-human-IgG1/3 antibody; and the second Western Blot comprising as step (d): washing the strips and performing a second incubation in a solution comprising FcR, as a supportive and confirmative experiment.

In other examples, steps (d) and (e) of Western Blotting above, may alternatively comprises washing the strips and performing an incubation in a solution comprising HRP conjugated with anti-human C3b, FcyRI, anti-human FcyRI, anti-Human Fab, anti-human IgA, anti-human IgG and/or antihuman IgM. In other examples, steps (d) and (e) of Western Blotting above, may alternatively comprises washing the strips and performing an incubation in a solution comprising with anti-human IgA, anti-human IgG and/or antihuman IgM.

In other examples, the method comprises using spectrophotometry, fluorescence microscopy, and/or other known techniques.

The isolated protein optionally comprises one or more of: complement binding proteins (e.g. C3b binding proteins), membrane proteins, outer membrane proteins (e.g. outer membrane protein A), immunoglobulin (IgG1 and/or IgG3) binding proteins, CR1 binding proteins, FcR (e.g. FcyRI) binding proteins, OprF, CipA, TSR domains, Extracellular complement-binding proteins, LPS-binding proteins, 0-N-acetyl-D-glucosamine, Protein A, Immunoglobulin-binding protein 1, teichoic acid, 0 somatic antigens, receptor binding proteins or surface proteins. The biomarkers, for example proteins, to be observed in the blood sample through binding with the isolated proteins of the electrode optionally comprise C3b, and/or IgG proteins. Optionally, wherein the isolated protein may be a receptor of the biomarker, wherein the biomarker is preferably C3b, IgG1 and/or IgG3.

In preferred embodiments, the proteins isolated from the bacterial cell wall, or the surface proteins of the pathogen strain, comprise one or more of: complement binding proteins (e.g. C3b binding proteins), immunoglobulin (IgG1 and/or IgG3) binding proteins, CR1 binding proteins, and FcR (e.g. FcyRI) binding proteins.

In some embodiments, the proteins isolated from the bacterial cell wall, or the surface proteins of the pathogen strain, comprise C3b binding proteins and optionally CR1 binding proteins. In some embodiments, the surface proteins or proteins isolated from the bacterial cell wall, comprise IgG1/ IgG3 binding proteins and optionally FcyRI binding proteins.

Optionally comprising selecting from the proteins identified to bind to one or more biomarkers according to the invention, proteins wherein the binding to the one or more biomarkers differs depending on whether the subject is infected or non-infected. Preferably, wherein the binding differs dependent on whether the subject is infected or non-infected with a bacterial pathogen strain corresponding to the bacterial pathogen strain the proteins are isolated from.

The method may further comprise: preparing a protein solution comprising the isolated proteins to be immobilized onto the electrode; and coating the protein solution on the electrode prior to immobilizing the protein to the electrode. The protein solution optionally comprising a mixture of the proteins and a carbonate buffer. Optionally, carbonate buffer or bovine serum albumin is used to prepare the protein solutions for the electrodes.

Optionally, further comprising washing the electrode with a phosphate buffer. Wherein washing may subsequently followed by drying of the electrode.

Immobilizing may comprise applying a potential difference across the electrode. For example, a voltage may be applied over a number of cycles having an increasing and/or fixed voltage. As a result of immobilizing the proteins may be fixed to the surface of the electrode and the electrode may be ready for testing. A plurality of conductive elements may be provided on the electrode to apply a potential difference across the electrode.

### Brief Description of the Drawings

Example embodiments are illustrated in the accompanying figures in which:
**Figure 1** illustrates a schematic of an example blood analysis electrode according to an embodiment of the present invention;
**Figure 2** illustrates a schematic of an example blood analysis system;
**Figure 3** illustrates a method of manufacturing an example blood analysis system and using the manufactured device to determine whether an infection is present according to an embodiment of the disclosure not part of present invention;
**Figure 4** illustrates a block diagram of a controller configured to determine an infection status of a person;
**Figure 5** is a circuit diagram of a controller configured to measure perform an electrochemical test on an electrode;
**Figure 6** shows a ROC curve analysis of the opsonic activity measuring C3b and Immunglobulin G comparison between, *A. baumannii* culture-positive and *A*. *baumannii* culture-negative groups.

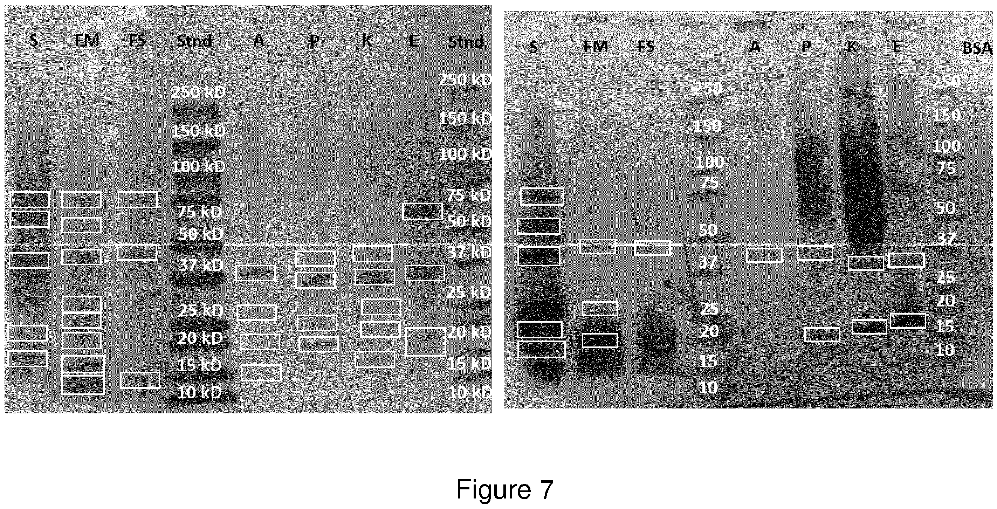

**Figure 7** shows Western Blots of opsonin binding surface proteins extracted from *S: Staphylococcus aureus,* Fm: *Enterococcus faecium;* Fs: *Enterococcus faecalis,* A: *Acinetobacter baumannii,* P: *Pseudomonas aeruginosa,* K: *Klebsiella pneumoniae,* E. *Escherichia coli.* The Western Blots shown were conducted using anti-human-C3b antibody and anti-human-lgG1/3 antibody.

In the drawings, like reference numerals are used to indicate like elements.

### Detailed Description

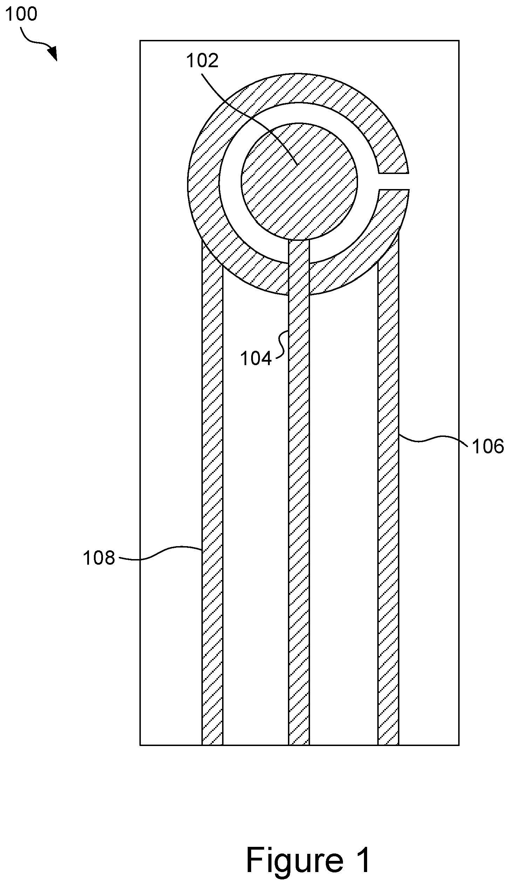

An example electrode 100 according to one embodiment is shown in Figure 1. An electrode such as a screen printed carbon electrode, for example a DropSens^{™} 110 electrode available from Metrohm^{™} , provide a suitable electrode for use with the below described embodiment.

The screen printed carbon electrode suitable for use comprises conductive elements based on carbon, gold, silver or carbon nanotubes. The general dimensions provided are 3.4 cm x 1.0 cm x 0.05 cm.

The electrode 100 comprises a working area 102 on the surface of a first end of the electrode 100 and a plurality of conductive elements 104, 106, 108. The conductive elements comprise a working line 104, a reference line 106 and a control line 108. At a second end of the electrode 100 is provided means for attaching the electrode 100, for example electrical contacts, to a controller, for example a device, for testing the electrode 100. The controller is configured to a pre-determined voltage to determine whether a subject has an infection or not. The reference line 106 and contacts at the second end of the electrode 100 may comprise silver or another conductive material.

The working area 102 is directly connected to the working line 104 and provides an area on the surface of the electrode 100 onto which a sample for testing, for example a blood sample, is deposited. The reference and control lines 108, 106 are in proximity to the working area 102 of the electrode 100 at the first end. The working, reference and control lines 104, 106, 108 extend from the second end of the electrode 100 (where an electrical connection between the lines and a controller is made) to the first, working end of the electrode 100 comprising the working area 102.

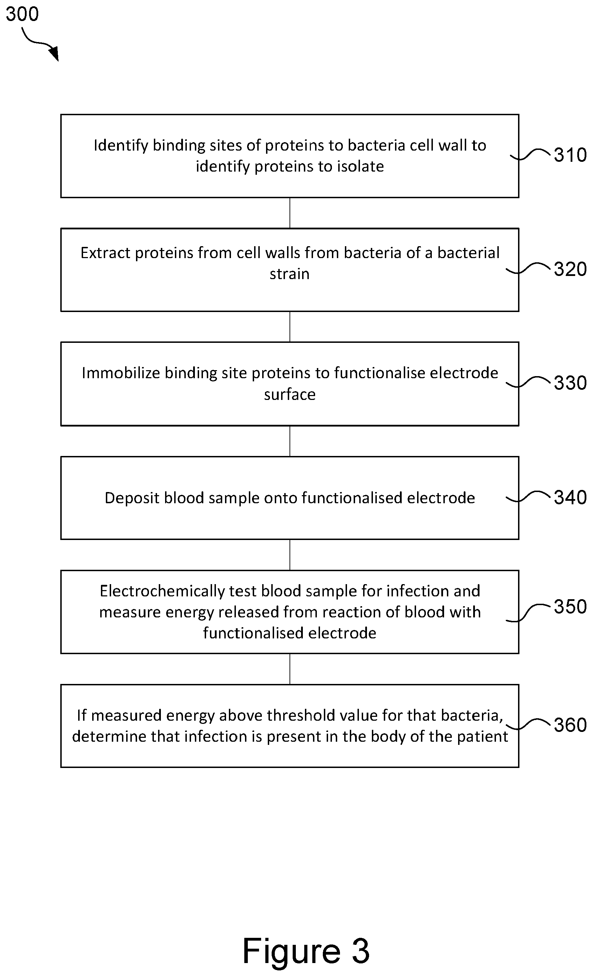

The working area 102 comprises a functionalised surface designed to interact with a blood sample disposed thereon. The working area 102 is functionalised using isolated proteins extracted from cell walls of a bacteria of the bacterial strain to be tested for. Isolated proteins, for example C3b binding proteins, outer membrane protein A, OprF, CipA, TSR domains, Extracellular complement-binding proteins, LPS-binding proteins, O-N-acetyl-D-glucosamine, immunoglobulin binding proteins, Protein A, Immunoglobulin-binding protein 1 or surface proteins are extracted from the bacterial cell walls. The bacterial strain that is used to functionalise the surface of the electrode 100 represents the bacterial strain of the infection to be tested for. A method of functionalising such a surface is described in more detail in relation to Figure 3 below.

The control line 108 carries the digital signals from a controller, for example comprising a central processing unit, CPU, to which the electrode is attached in use via the contacts at the second end of the electrode 100. The reference line 106 provides a fixed working voltage to the working area 102 of the electrode 100 in use. Electrochemical testing of the device can be performed using these lines to apply a potential difference across the working area 102. A potential difference may be provided to the electrode 100 during immobilization and/or during electrochemical testing of a blood sample. The controller controls the applied potential difference applied to the electrode 100.

The working line 104 sends signals to the controller resulting from interactions caused by binding between the functionalised working area 102 of the electrode 100 and the blood sample. The signals that are sent may comprise data. For example, data signals may comprise information concerning energy released by a biomarker, for example a protein, present in the blood sample that binds to an immobilized and/or isolated protein of an extracted cell wall on the surface of the electrode. The signals are generally indicative of a binding energy which can be used to determine the presence of the biomarker in the blood sample. The binding energy may be measured at the working area 102 as a voltage, for example. A signal is then sent to the controller via the working line 104.

The electrode 100 is configured at the second end to be compatible with and controlled by a controller, for example comprising a power supply, the controller is configured to provide the working area 102 of the electrode 100 with a current and/or voltage to activate the electrode 100 and perform blood analysis of a blood sample deposited thereon.

In use, a sample of blood to be tested is deposited on the functionalised area at the working area 102 of the electrode 100. A prescribed voltage cycle is provided to the working area 102 and the response of the blood sample deposited thereon is measured. In particular, the amount of energy released by biomarkers, for example proteins, in the blood sample that bind with the immobilized proteins of the extracted cell walls of the bacteria on the surface of the electrode is measured. From this measurement, it can be determined whether the person has been infected with the bacteria that is being tested for.

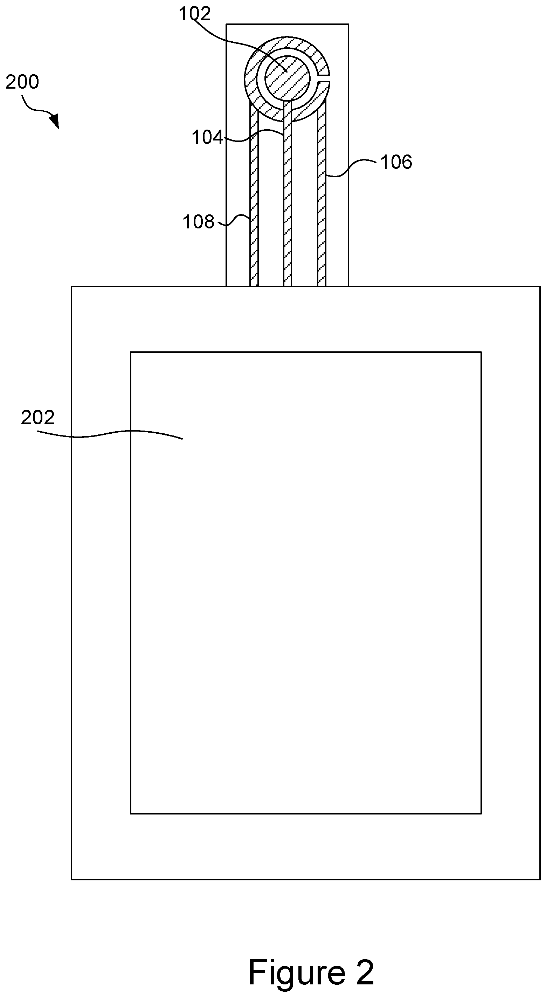

Threshold values of released energy may be applicable to individual bacterial strains as described in more detail in relation to Figure 2 and the Examples below. In order for a positive determination of the infection to be confirmed, the binding energy should provide a measured value above that of the threshold value for that particular bacterial strain.

Figure 2 illustrates an example of a user device or controller 200 to be used in conjunction with a functionalised electrode 100 as shown in Figure 1 and as described above.

The controller 200 comprises a user interface 220, which may comprise a screen, for example a touch screen. In some examples, the controller 200 may comprise a mobile phone, for example a mobile phone application which may be in communication with the electrode. In other examples the controller 200 comprises a purpose built device. The infection to be detected, or the bacterial strain associated with the infection are selectable from a list displayed on the user interface 220. Results of the determination are also presented at the user interface 220, for example on the screen.

The screen 220 provides a user with a visual representation of the resulting measured energy released by the functionalised electrode 100 and the blood sample. In some embodiments, the visual representation may simply state that an infection is present or not. In other embodiments, the visual representation may comprise a graphical representation of the results, i.e. the released energy, of the electrochemical testing performed on the electrode 100. The graphical representation may include a representation of a value, such as a threshold value. If the resulting measurements provided on the graphical representation are seen to exceed the threshold value, it can be determined that the bacteria, and hence the infection corresponding to that bacteria, are present.

The controller 220 is configured to control and receive electrical signals to and from the electrode 100. The controller 220 comprises modules suitable for applying a potential difference across the working area 102 of the electrode 100 and receiving a signal from the working area 102 in response to the applied potential difference via the working line 104.

From the received signals, the controller 220 determines whether the person, for example the patient, is infected or not infected. A threshold value is determined that the controller 220 uses to analyse the received data. Where received data indicates that there is an energy released by the interactions at the working area 102 of the electrode 100, the controller 220 determines whether this corresponds to an energy above the threshold value for that bacterial strain.

Threshold values are used to determine whether a bacterial strain has infected the body of the person who's sample is tested. The threshold value is unique to different bacterial strains because the biomarkers have unique conformational changes, and therefore unique binding energies, upon binding with the proteins from the cell wall of the bacterial strain. The threshold values for each specific bacterial strain can be determined experimentally, for example as detailed in the below examples. The threshold values can be learnt from a plurality of samples, for example blood samples, from various patients which are both known to be infected and uninfected. The determination of infection of the known patients can be determined using existing methods, for example the gold standard method which uses culturing of a patient sample and growing the pathogen. Each bacterial strain has a corresponding threshold value that is individually determined for that bacterial strain. Any bacterial strain can be used, but the threshold value for each bacteria should be determined individually. The types of bacteria which have been successfully used in an infection detection electrode comprise the following commonly seen infections in hospital environments, for example: *Klebsiella pneumoniae, Escherichia coli, Pseudomonas aeruginosa, Acinetobacter baumannii, Enterococcus faecalis, Enterococcus faecium, Staphylococcus aureus.* Other pathogenic or bacterial strains are compatible with the above described system, however, the specific parameters must be determined individually and are unique to each strain. Both gram positive and gram negative bacterial strains can be used in accordance with the above described electrode 100.

While not encompassed by the claims of the present disclosure, other products of the bacteria other than the above mentioned cell wall proteins of the bacteria may be used for a similar purpose. For example, the electrodes may be functionalised with ions and/or hormones produced by the strain of bacteria. The functionalised electrode provides a means for replicating a reaction that would naturally occur in the blood outside of the body. Simulating such a reaction outside of the body provides a non-invasive, quick and accurate determination of whether bacteria has infected the body of the patient or not.

The electrode 100 may be in direct contact with the controller 200 as shown in Figure 2, however, in other embodiments, the electrode may be in indirect connection with the controller, for example via a wireless or wired connection. The controller 220 could be configured to control a power supply connectable to the electrode 100 in, for example, a different location or room to the controller 220.

The controller 220 may be further configured to test a plurality of electrodes 110 at once, and may comprise a number of connectable locations, either at the controller itself or at locations remote to the controller, configured to receive an electrode 100.

Figure 3 illustrates a method of manufacturing an example blood analysis system and using the manufactured device to determine whether an infection is present in a patient or not according to an embodiment of the present disclosure not part of the present invention.

At step 310, proteins capable of binding to one or more biomarkers, for example C3b, IgG and/or FcyRI, are identified such that they can be isolated from the extracted bacterial cell walls and immobilized on the surface of the electrode. This step is a precursor to the manufacturing of the electrodes and provides the knowledge required to determine the correct protein to extract from bacteria of the bacterial strain. The selection of proteins, or isolated proteins, is important to ensure the biomarkers in the blood sample can bind to the electrode. Proteins capable of binding to one or more biomarker can be identified, for example, as follows:

### Collection and culture of bacterial strains

The reference strain of the pathogenic strain from which the proteins are to be isolated may be used for protein profiling. For example, the reference strain (standard organism) of *Klebsiella pneumoniae, Escherichia coli, Pseudomonasaeruginosa, Acinetobacter baumannii, Staphylococcus aureus, Enterococcus faecalis,* and/or *Enterococcus faecium* are used.

Working cultures are prepared, for example, by inoculating one single isolated colony in 10 ml of brain heart infusion (BHI) broth, and incubating overnight at 37 °C with shaking at 200 rpm in an orbital shaker. 200 µl of the samples obtained as a resultofincubation are transferred to a new flask and diluted with fresh BHI broth to give 0.2 absorbances at 600nm. The samples are left to incubate at 37 degrees 200 rpm for at least 30 hours. OD measurements are made at two-hour intervals during incubation. Incubation is stopped after 5 consecutive identical results have been measured. Before consecutive identical results, the OD value is taken as the mid-log-phase OD value for the pathogen in question.

| Pathogen | Abosorbance Value | OD Value | Incubation Time |
|---|---|---|---|
| *Acinetobacter baumannii* | 600 nm | ~ 2 | 22 hours |
| *Klebsiella pneumonia* | 600 nm | 1,-1.2 | 21 hours |
| *Staphylococcus aureus* | 600 nm | 0.8-1 | 10 hours |
| *Escherichia coli* | 600 nm | 1-1,2 | 18 hours |
| *Enterococcus faecium* | 600 nm | 0.8-1 | 14 hours |
| *Enterococcus faecalis* | 600 nm | ~ 0.8 | 4 hours |
| *Pseudomonas aeruginosa* | 600 nm | 0.6-1 | 12 hours |

### Protein extraction

After incubation of bacteria until the mid-log phase, bacterial samples are transferredto 50 ml falcon tubes and cells are centrifuged at 21.000 x g for 30 min. The pellet is resuspended in 8 ml of 0.01 mol/I HEPES buffer (pH 7.4). Bacterial cells are disrupted by vortexing with glass beads (150-212 microns) for 1.5 h. Unbroken cells are removed by centrifugation at 5000 x g, 4 °C for 15 min. Cell lysis is monitored using the Gram stain method. The supernatant obtained is centrifuged at 200,000 x g for 60 min.

The pellets from Staphylococcus aureus, Enterococcus faecalis, and Enterococcus faecium are suspended with 100ul of sterile distilled water. The pellets from Klebsiella pneumonia, Escherichia coli, Pseudomonas aeruginosa, Acinetobacter baumannii are mixed with 10 mM HEPES (pH 7.4) containing 1-5 ml of 1% sarcosyl, and incubated for 15 min. Then, the samples are centrifuged at 200,000 x g for 60 min, and the pellet is diluted with 100ul sterile distilled water.

Protein concentration may be determined using the following protocol: 1 ml of protein assay stock solution is mixed with 4 ml of Phosphate Buffered Saline (PBS), pH 7.4 to make the working solution. In a microtiter plate, 200 µl of the solution is pipetted into each well and 10 µl of protein sample and also protein standard (Bovine Serum Albumin standards of 50 to 500 µg) is added. The solution is mixed thoroughly and absorbance is measured at A595 nm using the microplate reader. Relative measurement of protein concentration is determined by comparing the protein absorbance to a standard curve graph. The extracted samples are stored at -80°C for further use to determine the presence or location of the specific protein in it.

### SDS-PAGE

Sodium dodecyl sulfate-polyacrylamide gel electrophoresis (Discontinuous SDS-PAGE) is performed to analyze bacterial proteins, to determine surface protein profiles of Klebsiella pneumoniae, Escherichia coli, Pseudomonas aeruginosa, Acinetobacter baumannii, Staphylococcus aureus, Enterococcus faecalis, and Enterococcus faecium based on differences in their molecular size.

Protein samples were diluted 1: 1 in sample loading buffer, wherein 30ml of sample loading buffer comprises: 0.5M Tris-HCl Ph: 6.8: 3.75 ml, 50% Glycerol: 15.0ml, 0.1% Bromophenol blue: 0.3ml, 10%SDS: 6.0 ml, ddH20: to 30 ml, Beta-Mercaptoethanol : 50 µl to 950 µl sample buffer. Samples were heated with a dry plate at 95°C for 5 minutes. Running buffer (30.30 g Tris base, 144.10 g glycine and 10.0 g SDS) was dissolved in 1000 ml distilled water.

A strip of gel is loaded with prepared bacterial cells and the proteins are separated on the gel. Each lane of the gel was loaded with 15 µg of the protein sample, and 3 µL of the marker was also added. Proteins were separated on polyacrylamide gel by applying 25 mA for 30 minutes at 90 voltage. The gel was run until the pre-paint flows from the bottom of the gel. Optionally, the resulting proteins are suspended with sample buffer containing SDS and β-mercaptoethanol.

The proteins are observed with SDS-PAGE gel stained with Coomassie blue with the following protocol: the gel was put in 20 ml of Coomassie blue staining solution (Coomassie blue: 0.05 g, Asetic acit: 1 ml; Methyl alcohol:20 ml, ddH20:29 ml) and shake for 20 minutes, shaking gently. Then the gel was put in 15 ml of destaining solution (Aesthetic Acid: 10ml, Methyl alcohol : 20ml, ddH20: 29ml) two times for 15 min.

### Western Blot

Proteins that become separated by the SDS-PAGE are electroblotted from the gel to the membrane (optionally a nitrocellulose membrane or PVDF membrane) by electrophoretic transfer. The purpose was to determine which, if any, proteins of *Escherichia coli, Pseudomonas aeruginosa, Acinetobacter baumannii, Staphylococcus aureus, Enterococcus faecalis,* and *Enterococcus faecium,* are both antigenic and specifically recognized by C3b, IgG1 and IgG3 in serum from humans carrying infections with these bacteria.

The membrane is incubated with 5% of skim milk in Tris Buffered Saline with Tween 20 (TBST) for 1 h at room temperature to block the nonspecific protein binding sites. The membrane is washed with TBST 5 times for 5 minutes on shaker, cut into strips and incubated at 4C on shaker for overnight with 1: 100 dilutions of human serum (infection positive and infection negative serum are used separately) as the first antibody. After primary antibody incubation, the strips were washed with TBST 5 times for 5 min. The strips then were incubated with mouse anti-human IgG1, anti-human IgG3, and antihuman C3b for overnight at 4C with gentle agitation. The strips were washed with TBST 5 times for 5 minutes. Then the strips were incubated with conjugated antibody for 1 hour at room temperature. The strips were washed with TBST 5 times for 5 minutes. The strips were then incubated with conjugate spesific substrate for 30 min. The enzyme reaction was stopped by rinsing the strips with distilled water. The results were visualized.

The Western blot was repeated with CR1 and FcR1 y as the secondary antibody instead of anti-human C3b and anti-human IgG1 / IgG2, respectively, to observe the same protein profiles, provided the other steps were the same.

Another method of Western blot may comprise one or more of the following steps: blocked nitrocellulose membranes are cut into strips and incubated with human serum (infection positive and infection negative) dilutions; after the primary antibody incubation, the strips are washed and the membrane is incubated with HRP conjugated with antihuman C3b, FcyRI, anti-human-FcyRI, anti-human Fab, anti-human IgA, anti-human IgG and antihuman IgM. After incubation, the strips are washed and incubated with HRP-conjugated substrate. The strips are then rinsed with distilled water to prevent the enzyme reaction. Protein bands detected by the HRP catalysed reaction are displayed on the image analyser. Proteins capable of binding to C3b, and/or IgG are selected for immobilization to electrodes. Glycoprotein staining is performed using the glycoprotein staining kit. The trypsin digestion test is performed to determine if the main component of the band is a protein. Protein concentration is determined using a protein assay kit as commonly known and used in the art. The assay is performed three times to obtain a sufficient protein concentration. It will be appreciated that the above method describes an example method and that the process is not limited to the method as described above.

Proteins identified using the above method include C3b binding proteins Immunoglobulin (IgG1/IgG 3) binding proteins, CR1 binding proteins or FcR (e.g. FcyRI) binding proteins.

It will be appreciated that other proteins may bind to C3b, IgG (e.g. IgG1 and/or IgG3) and that these proteins would also be suitable for use with the functionalised electrode. It will also be appreciated that other biomarkers and corresponding proteins in the cell wall that said biomarkers bind to may be used in the same manner as disclosed herein.

At step 320, the method comprises extracting the proteins from the cell walls of the bacteria. Step 320 is a manufacturing step based on the research step 310, and is required to functionalise the surface of an electrode.

According to the results of Western Blot, C3b binding proteins and IgG1/IgG3 binding proteins may be purified with one or more purification steps selected from: gel filtration, ion exchange filtration, immunoprecipitation, ultrafiltration and dialysis.

The above processes are well known and may be substituted for other suitable methods achieving the same resulting isolation and/or purification of pathogen surface proteins, or achieving the same resulting extraction of the cell walls of the bacteria. The method is therefore not limited to the above described method.

The method may alternatively, comprise sequencing C3b binding proteins and IgG1/IgG3 binding proteins identified by the Western Blot, and extracted using the methods described above.

Optionally, the protein sequencing comprises one or more of: dissolve samples containing ~200ug proteins in 50 mM ammonium bicarbonate; centrifuge at 12,000 g at 4 °C for 10 min; discard supernatant and add 200 µL of 50 mM ammonium bicarbonate followed by centrifugation to collect the pellet and repeat twice; reduce the sample by 10 mM DL-dithiothreito at 56 °C for 1 hour; alkylate the sample by 20 mM iodoacetamide at room temperature in dark for 1hour; centrifuge at 12,000 g at 4 °C for 10 min and wash the pellet with 50 mM ammonium bicarbonate; add 100 µL of 50 mM ammonium bicarbonate and free trypsin (or other enzymes with their buffers) into the protein solution at a ratio of 1:50 at 15 ng/ul concentration, and the solution is incubated at 37 °C overnight to get the small peptides; centrifuge the mixture at 12,000 g at 4 °C for 10 min and collect the pellet; add 100 µL of 50 mM ammonium bicarbonate and centrifuge at 12,000 g at 4 °C for 10 min and collect the pellet, and then repeat the wash step once; lyophilize the extracted peptides to near dryness, resuspend peptides in 2-20 µL of 0.1% formic acid; apply the 5 ul sample with nano liquid chromatography by reversed-phase ReproSil-Pur C18-AQ resin column; detect the sample by Obitrap Q Exaxtive HF mass spectrometry with 60000 at 400 m/z resolution; analyse the MS data with PEAKS software and detect the sequence of protein.

Optionally, the method may further comprise expression or synthesis of the sequenced proteins, for example using techniques known in the art.

At step 330, the proteins capable of binding one or more biomarker (for example C3b binding proteins and IgG1/IgG3 binding proteins) are immobilized on the surface of the electrode to functionalise it. This step completes the manufacturing and functionalisation of the electrode such that it can be subsequently used to determine whether a patient has been infected or not by measuring the response to their blood sample.

Proteins extracted or isolated from the bacterial cell wall, or the surface proteins of the pathogen strain, preferably capable of binding one or more biomarker (C3b binding proteins and IgG1/IgG3 binding proteins), are immobilized onto the surface of the electrode, for example screen printed carbon electrodes such as a DropSens^{™} 110. A device such as a potentiostat device can be used to immobilize the proteins onto the surface of the electrode. An example method of immobilizing the proteins comprises the following steps:
Firstly, 1 McFarland protein solutions are prepared with a carbonate buffer. 100µl of proteins from 1 McFarland suspension are then taken and deposited on the surface of the working are on the surface of the electrode. A device, for example a potentiostat device, is then used to immobilize the proteins on the surface of the electrode to functionalise the working area. Example values used to immobilize the proteins on the working area of the electrode using the potentiostat device comprise the following: cyclic voltammetry, voltage range: -1/+1, scan rate: 20-100 mV/s, step size: 1-5 mV, repeated number of cycles: 10-100, maximum current: 1000 mA. It will be appreciated that variations to the above method can be used to achieve immobilization of the proteins; any known method may be used.

The surface of the electrode that has been functionalised is subsequently washed with a phosphate buffer and dried. After drying, the electrode is ready for use with the controller to measure and/or determine if there are any infections present in the blood sample provided by a patient.

Before step 340 and after step 330, the electrode may be calibrated. Calibrating the electrode ensures that the signals that are received at the controller are representative of the reaction at the working area of the electrode.

At step 340, a blood sample is deposited onto the functionalised surface of the electrode. Step 350 is a method step, the method being undertaken to determine a result for a specific patient.

To test a blood sample, a 100µ1 of blood sample from patient is disposed on the functionalised electrode surface at the working area. After preparing the electrodes with the proteins from the cell wall of the bacteria and depositing a sample of blood onto the working area of the electrode, the electrode is electrically connected to the device or controller configured to receive the electrode. The device is configured to perform an electrochemical test on the electrode to determine presence or absence of an infection in the body of the person that provided the sample.

At step 350, electrochemically test the blood sample and measure energy released from the sample deposited on the functionalised electrode. The controller is configured to control the electrochemical test. Step 350 is a further method step performed for a specific patient's blood sample.

A binding energy of C3b and/or IgG proteins (e.g. IgG1, IgG3) in the blood sample binding with the functionalised electrode is released if the patient has an infection (e.g. bacterial infection) corresponding to the bacterial strain coated on the electrode. The binding of the proteins in the blood sample with the electrode can be achieved by subjecting the electrode to a voltage, for example between -1mV and +1mV for 300 to 1000 seconds, for example at -0.5mV for 300 seconds. In the case that patient is not infected, there will be less energy released, for example energy that is under the measured threshold energy for that bacteria, and the data sent to the controller will reflect this. On the other hand, if the infection is present, binding between the proteins will be evident by the determination of a released energy, in particular of released energy over the threshold for that bacterial strain. Signals comprising released energy data are sent to the controller for determination of presence or absence of the infection.

At step 360, if the measured energy is above the threshold value for the bacteria that is being tested, it is determined that the infection is present in the body of the patient. The controller receives signals comprising data from the working area of the electrode and compares it to the threshold value for that bacteria.

As the controller receives data from the electrode, it may plot a result of the energy released against time.

An additional step, not illustrated in Figure 3, may comprise transmitting the resulting determination to the patient or a healthcare official of the patient directly, for example, via an electronic message (e.g. via email or text), or for example through an electronic application such as a downloadable mobile phone application. The patient may be able to receive their results through such a mobile application. Alternatively, the device may be controlled by a mobile phone application. The controller may be additionally configured to write a prescription for an antibiotic to cure the patient.

Treatment of the infection can subsequently be prescribed using antibiotics for the infection detected. Antibiotics can be effective for different strains of the bacteria that were determined to be present as a result of the electrochemical test performed on the sample. A treatment profile may be determined by the controller.

Identifying a response (e.g. an immune response) against a pathogenic strain, by measuring the binding energy of biomarkers of response (e.g. immune response), correctly detects an infection with over 90% accuracy in samples of 450 different people. For C3b and IgG, it is hypothesised that measurement of a specific binding energy of C3b and/or IgG, reflects whether the C3b and/or IgG have previously bound (opsonised) the proteins of the cell wall of the bacteria strain, and therefore reflects whether the patient is infected with the bacteria strain or not. The method has been compared to the conventionally used culturing method with the results that are shown below in the detailed examples.

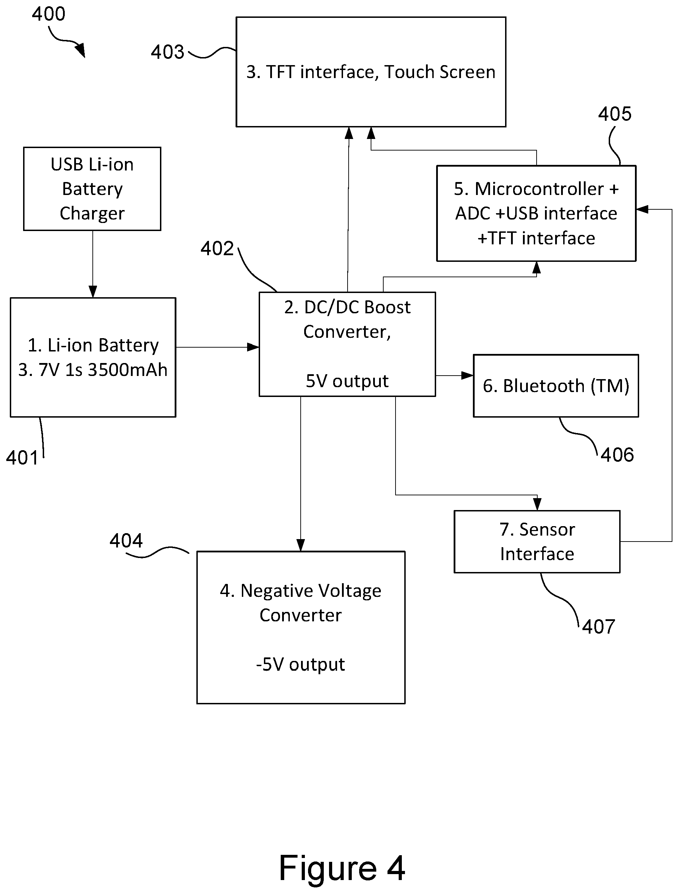

Figure 4 illustrates a block diagram of a controller configured to determine an infection status of a person.

The device is supplied with energy at 401. For example, by a lithium ion battery (3.7 V, 3500 mAh battery), for example a rechargeable battery, and is provided with a circuit to charge the battery. Charging via USB is also a possibility.

The battery voltage is converted at 402 to the operating voltage of the display (for example to achieve a 5V output). Converted energy feeds a microcontroller, Bluetooth^{™} for wireless data transfer, and the sensor circuit including the electrode 100.

The touch screen is supplied by the converted energy at 403, obtained in Step 402. At the same time, communication with the microcontroller is via UART and provides a serial communication.

At 404, the 5V obtained in Step 402 is converted to -5V at the same time. This dual voltage is used to feed the circuit on the sensor side.

A processor is used at 405 as a microcontroller (for example, ATMega2560^{™}). The 10 bit analog input of the processor is used to read the analog value from the sensor side comprising the electrode and sample deposited thereon.

At 406 there is a Bluetooth^{™} interface on the controller for wireless transfer of data over a short-range communication.

There is provided a sensor circuit of the device 407. The user selects the bacteria from a list displayed on the screen. Here the voltage value of the threshold is embedded in the device for the selected bacteria as determined experimentally.

When the bacteria is selected from the screen in 407, the microcontroller sends a command or signal to set this corresponding voltage as the threshold voltage. This is adjusted on the sensor side, e.g. at the electrode. At this voltage, the bacteria-specific reaction begins and the current is released as a result of the reaction. The released current is measured by the microcontroller.

The binding energy is detected by monitoring the peak current (typically acquired at a working potential of 0- (-1) V vs. Ag/AgCl) of the voltammetric or amperometric signal. Differential pulse voltammetry, impedance spectroscopy, linear sweep voltammetry, chronoamperometry, and cyclic voltammetry can be used to measure the peak current.

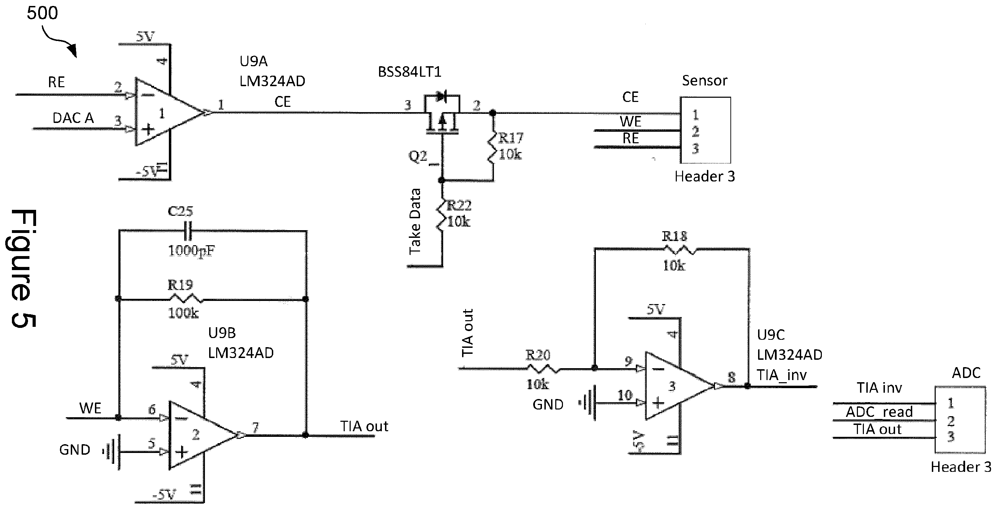

Figure 5 is a circuit diagram of a controller configured to perform an electrochemical test on an electrode. It shows example circuitry that can be used to perform the steps as detailed above in relation to measurement of a sample deposited on an electrode.

The biological process that can be used to determine the presence or absence of an infection of a patient can be described as follows. If a bacterial strain is, or has been, present in the patient, an immune response is generated by the infected patient. Examples of immune responses associated with bacterial infection include complement pathway, opsonisation, and macrophage activation. Biomarkers may include, for example, proteins, peptides or protein complexes wherein the expression, oligomerisation and/or conformation of said biomarker is upregulated by an immune response associated with a type of infection (e.g. bacterial infection). These responses are characterised by biomarkers present in the patient's blood sample which are changed by their interaction with a bacterial cell present in the infected blood. Examples of biomarkers, for example proteins, upregulated by an immune response include C3b, and IgG (e.g. IgG1 and IgG3). The presence of such a biomarker, or biomarkers, in the blood is therefore indicative of the presence of a infection (e.g. bacterial infection) so detecting these biomarkers, for example to the pathogen, is an effective way of determining that a patient is infected or not. The bacteria itself is not required to be directly detected, rather the immune response of the patient. If there is an infection, C3b and/or IgG bind to the proteins isolated from the cell wall of the bacterial strain and release a specific binding energy. This binding energy is dependent on a conformational change conferred by previous binding of the C3b and/or IgG (e.g. IgG1 and IgG3) to the infectious bacteria.

C3b, IgG (e.g. IgG1 and IgG3), CR1 and FcyRI are biomarkers of infection (e.g. bacterial infection) present in the blood and capable of binding to a protein isolated from a bacterial cell wall. Determination that the biomarkers have the capability to bind to a bacterial cell wall protein was assessed by spectrophotometery, fluorescent microscopy and electrochemical readings. Research has been performed to confirm proteins capable of binding to one or more biomarkers, for example C3b and IgG (e.g. IgG1 and/or IgG3) proteins, in particular proteins isolated from the bacterial cell wall. Evidence of this research is given in the below Examples.

The proteins from the cell wall of the bacteria used to functionalise the electrode comprise proteins that bind to the biomarker, for example C3b and IgG proteins, present in the patient's blood sample. Research has shown that the proteins that can be isolated from the bacterial cell wall that bind C3b or IgG comprise one or more of: C3b binding proteins, outer membrane protein A, OprF, CipA, TSR domains, Extracellular complement-binding proteins, LPS-binding proteins, 0-N-acetyl-D-glucosamine, immunoglobulin (e.g. IgG1, IgG3) binding proteins, CR1 binding proteins or FcR (e.g. FcyRI) binding proteins, Protein A, Immunoglobulin-binding protein 1 or surface proteins. One or more of these proteins are immobilized on the surface of the electrode to prepare the electrode for use.

A binding occurs at a site of the activated electrode where an isolated protein from an cell wall extracted from the bacteria binds with a biomarker, for example a protein (such as C3b or IgG), present in the blood sample. The binding produces an energy, for example a binding energy, under electrochemical testing of the electrode that can be measured. Measuring a resulting binding energy is therefore indicative of the specific immune response of the patient, which is consequently indicative of a presence of the bacterial strain in a patient. It can therefore be determined that the bacteria, and in turn the associated infection, are present and that the patient is infected if a binding energy is measured. Absence of a reaction, for example a marked reaction, between the functionalised electrode and the blood sample is correspondingly indicative that a patient is not infected by the bacteria that are being tested for.

Comparative Example 1 Proteins specifically binding C3b were shown to demonstrate altered binding to a patient blood sample dependent on whether the sample was taken from a non-infected or infected patient, and therefore proteins capable of binding C3b are suitable exemplar proteins isolated from a bacterial cell wall for functionalisation of an electrode according to an embodiment of the disclosure not part of the invention. 152 patient blood samples were incubated with proteins isolated from the cell wall of A. baumannii bacteria, wherein the proteins specifically bind to C3b. Fluorescence microscopy was used to evaluate binding of the proteins isolated from the A. baumannii to the blood in each of the 152 samples. For each sample, 100 sites were monitored for a colour change indicative of protein binding. A threshold value for the number of sites, out of 100, where a colour change must occur to correctly classify a patient as having an A. baumannii infection, with the desired sensitivity and specificity was determined by comparison to commercial methods of bacterial cell culture to detect an A. baumannii infection in the same 152 blood samples.

The sensitivity for detecting an infection using this method was found to be 100% at a threshold of >=79, but drops off if the threshold is increased above 79. The sensitivity is such that it does not matter at what stage of infection the patient is. For example, the system can determine that a patient is infected whether they have had the bacteria in their system for not long enough to show symptoms of the infection as well as those who have clear symptoms.

The opsonic activity of different groups of people was measured. The data is detailed below. The groups had resulting opsonic activity: group 1 comprising 50 patients infected with Acinetobacter baumannii had an average opsonic activity of 92.3%; group 2 comprising a positive control group of 30 patients had an average opsonic activity of 97.87%; group 3 comprising 12 patients infected by another pathogen had an average opsonic activity of 38.42%; group 4 comprising 30 non-infected hospital patients had an average opsonic activity of 45.83%; and group 5 comprising 30 healthy people had an average opsonic activity of 37.2%. A threshold sensitivity level of >=79 was used.

The following assays were used to determine this data.

### Assay one:

1. 100 µl suspension of Acinetobacter baumannii, 100 µl serum samples from people who were tested, and 200 µl of pure leukocytes were put in a sterile tube. A control tube was prepared with PBS instead of people's serum.
2. Incubated for 10 min at 37 degrees Celsius. After, it was put on ice to stop the reaction.
3. Washed with wash buffer, then centrifuge for 5 minutes, followed by decanting the supernatant (3 times).
4. 200 µl of Bacteria Counting Kit Mix was added and incubated for 10 minutes on ice (light protected in the ice bath).
5. Measure the bacteria bonded leukocytes within 30 minutes under fluorescent microscopy, also and flow cytometry.
6. Using fluorescent microscopy, the bacteria bonded leukocytes were measured from 100 leukocytes.
7. Using flow cytometry, the density of the bacteria in the sample was determined from the ratio of bacterial signals to microsphere signals in the cytogram.

### Assay two with the same samples:

1. 100 µl suspension of Acinetobacter baumannii, 100 µl serum samples from people who were tested, and 200 µl of pure leukocytes were put in a sterile tube.
2. Incubated for 10 minutes at 37 degrees Celsius then put on ice to stop the reaction.
3. Washed with wash buffer, centrifuged for 5 minutes, and then decanted the supernatant (3 times).
4. 50 µl of the mixture was put on a lam and prepared peripheral smear.
5. The mixture in the tube was stained with Giemsa.
6. Measured the bacteria bonded leukocytes from 100 leukocytes und er flourescence microscopy.

These results represent that the bacteria-specific opsonic activity of the samples taken and show that it is possible to diagnose an infection presence or absence compared with the standard culturing results. Different people have different opsonisation based on the presence of infectionand also the bacterial strain.

Opsonisation involves the binding of an opsonin, C3b, or an antibody, IgG, to an epitope on a pathogen. C3b and IgG1-IgG3 serve as an opsonin. After C3b and IgG have bound to the surface of a pathogen, the phagocyte receptors recognise this as a signal for phagocytosis. Antibodies that facilitate phagocytosis of microbes are called opsonin (C3b, IgG1, IgG3) and this phenomenon is called opsonisation. Antibody opsonisation contributes to phagocytosis and destruction of bacteria. Phagocytosis is not seen in the blood of uninfected people, while people with a positive phagositic opsonic index are considered to have an immune response or infection.

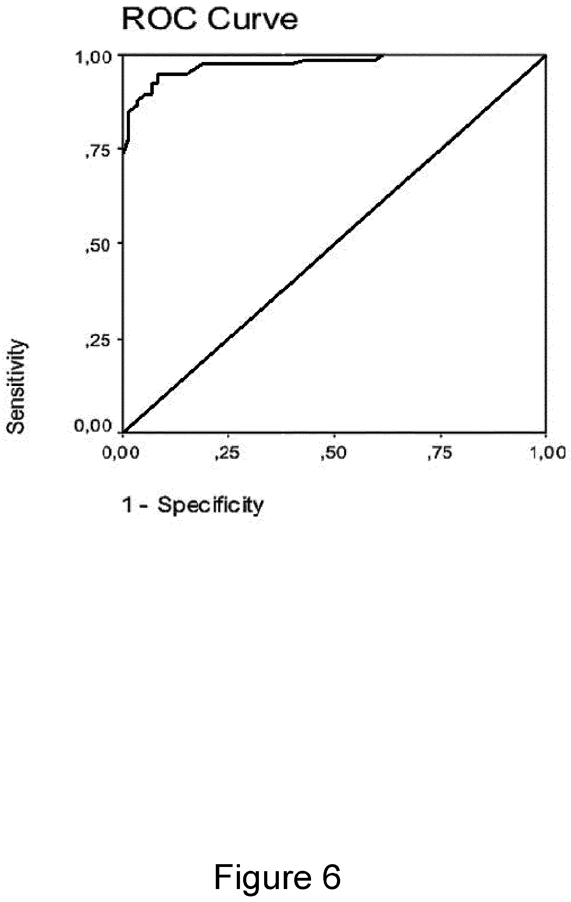

Figure 6 illustrates a receiver operating characteristic curve, or ROC curve, which is a graphical plot that illustrates the diagnostic ability ofa binary classifier system as its discrimination threshold is varied. The ROC curve analysis shows that the opsonic activity (with measuring C3b and Immunglobulin G) comparison between, *A. baumannii* culture-positive and *A*. *baumannii* culture-negative groups. Fluorescence and lighter microscopy assays were used together to obtain the results. There was one group of healthy people, one group of non-infected hospital patients, and three groups of patients with a known infection status as detailed above.

The ROC curve analysis shows the opsonisation test comparison between A. *baumannii* culturepositive and *A*. *baumannii* culture-negative groups.Results between the two groups have 95% sensitivity and 91.7% specificity. The positive predictive value of the test calculated as 92.7%, the negative predictive value calculated as 94.3% and the validity of the test calculated as 93.4%.

**Table 1: Distribution of Acinetobacterbaumannii positive and negative patients according to the opsonic activity test threshold.**

| **True** | ***Acinetobacter* (*+*)** | | ***Acinetobacter* (-)** | | **Total Test** | |
|---|---|---|---|---|---|---|
| | **Patients** | | **Patients** | | | |
| | n | % | n | % | n | % |
| Positive | 76 | 95 | 6 | 8 | 82 | 54 |
| Negative | 4 | 5 | 66 | 92 | 70 | 46 |

In this distinction, the area under the ROC curve of the opsonisation was 0.976, which was found to be an excellent test method in the test A category. When the culturing method was taken as a reference test, opsonisation values of four patients from Acinetobacter positive-culture patients group we found as negative as shown in the above Table 1. These patient's cultures were found to be positive because of Acinetobacter baumannii colonisation.

Opsonisation values of six patients from *Acinetobacter* negative-culture patients group were found to be positive, although they should have been below the threshold value their results were found to be above the threshold value. These patient's cultures could be negative because of antibiotic usage of the patients, taken in order to combat the infection.

**Table 2: ROC curve analysis results.**

| **Parameters** | **Values** |
|---|---|
| Sample Size | 152 |
| ROC Area | 0.976 |
| Standard Deviation | 0.011 |
| Confidence Interval 95% | 0.995 - 0.997 |
| Cut off | ≥ 79 |
| Sensitivity | 95% |
| Specificity | 91.70% |
| PPV | 92.70% |
| NPV | 94.30% |
| Test Validity | 93.40% |

The ROC Curve analysis shown in Figure 6 and detailed in the above Table 2 shows that the positive predictive value (PPV) of opsonic activity test in VAP was 92.7%, the negative predictive value (NPV) was 94.3% and the validity of the test was 93.4%.

Comparative Example 2 Proteins specifically binding C3b and/or IgG were shown to be suitable exemplar proteins isolated from a bacterial cell wall for functionalisation of an electrode according to an embodiment of the disclosure not part of the invention. Significantly, binding of bacterial proteins specific for C3b and/or IgG could be used to determine infections of 7 different bacterialstrains using said bacterial proteins isolated from the corresponding bacterial strain cell wall.

The method used to detect an antibody response to a patient's sample was performed as follows:
1. Cover the microplate (96 wells) surface with bacterial cell wall proteins.
2. Incubate at 4 degrees Celcius for one night.
3. Wash the microplate with wash buffer to eliminate nonbinding cells.
4. Put in each well of the microplate 100 µl of infected and non-infected patient serums-sterilized PBS and incubate for 15 minutes at 37 degrees Celcius.
5. Wash the microplate with wash buffer to eliminate nonbinding cells.
6. Put 100 µl anti-human Fc gamma receptor 1 and CR1 in each well of the microplate and incubate for 15 minutes at 37 degrees Celcius.
7. Wash the microplate to eliminate nonbinding cells.
8. Put 100 µl PNPP (p-Nitrophenyl Phosphate, Disodium Salt) in each well of the microplate and wait 20 minutes in a dark space.
9. Put 100 µl sulfuric acid in each well of the microplate to stop the reactions.
10. Measure the colour density at 405 nm with a spectrophotometer to measure the opsonisation of the bacterial cell wall proteins.

Spectrophotometry was used to determine binding of proteins specific to C3b and/or IgG isolated from the bacterial cell wall, which were coated onto a plate, to a blood sample which was deposited on the plate surface. The colour density at 405 nm, was used to measure the binding of said proteins to C3b and IgG and the opsonisation value.

The results are shown in the below tables. In the below Table 3, group 1 represents A. *baumannii,* group 2 represents *K. pneumoniae,* group 3 represent *E. coli,* group 4 represents *P*. *aeruginosa,* group 5 represents *E*. *fecilas,* group 6 represents *E*. *faecium* and group 7 represents *S*. *aureus.* For each of the groups, results were compared to bacterial growths of the same patients assessed using the gold standard of culturing a patient sample and growing the pathogen to determine presence of the bacteria, 1 indicating an infection was found (i.e. bacteria detected by the gold standard method) and 0 indicating no bacteria of that strain were detected by the gold standard method). The sample type of these gold standard bacterial growths is represented by 2 for a sterile body fluid, 1 for other samples which may have some contamination, and 0 where no bacterial growth sample was taken for cell culturing. N is the number of patients and the percentage shows the percentage that n represents out of the total n for that row.

**Table 3: Details of tested samples for each group according to sex presence of bacteria and sample type.**

| | | **Group 1** | | **Group 2** | | **Group 3** | | **Group 4** | | **Group 5** | | **Group 6** | | **Group 7** | |
|---|---|---|---|---|---|---|---|---|---|---|---|---|---|---|---|
| | | n | % | n | % | n | % | n | % | n | % | n | % | n | % |
| **Sex** | 0 | 32 | 50,8 | 20 | 55,6 | 20 | 42,6 | 20 | 42,6 | 11 | 45,8 | 27 | 52,9 | 14 | 46,7 |
| | 1 | 31 | 49,2 | 16 | 44,4 | 27 | 57,4 | 27 | 57,4 | 13 | 54,2 | 24 | 47,1 | 16 | 53,3 |
| **Bacterial Growth** | 0 | 42 | 66,7 | 24 | 66,7 | 18 | 38,3 | 19 | 40,4 | 11 | 45,8 | 21 | 41,2 | 21 | 70,0 |
| | 1 | 21 | 33,3 | 12 | 33.3 | 29 | 61.7 | 28 | 59,6 | 13 | 54,2 | 30 | 58.8 | 9 | 30,0 |
| **Sample Type** | 0 | 20 | 31,7 | 12 | 33,3 | 12 | 25,5 | 7 | 14,9 | 9 | 37,5 | 17 | 33.3 | 15 | 50,0 |
| | 1 | 16 | 25,4 | 4 | 11,1 | 14 | 29.8 | 16 | 34,0 | 6 | 25,0 | 11 | 21,6 | 8 | 26,7 |
| | 2 | 27 | 42,9 | 20 | 55,6 | 21 | 44,7 | 24 | 51,1 | 9 | 37,5 | 23 | 45,1 | 7 | 23,3 |

The below tables 4-5 illustrate the resulting opsonisation values of blood samples from patients determined from a bacterial growth sample to be infected or non-infected with the bacteria. The number of samples that were tested for each bacterial group is given in the "Number" column. The average opsonisation value is shown for that number of samples, and the standard deviation is also calculated. Table 4 shows the results for the positive and negative results combined. The data used to determine these Tables is detailed below.

**Table 4: Results of average opsonisation values at an optical density of 405nm.**

| | **Number** | **AVG** | **SD** |
|---|---|---|---|
| *A. baumannii* | 63 | 1,0647 | ,6351 |
| *K. pneumoniae* | 36 | ,8384 | ,4925 |
| *E. coli* | 47 | 7735 | ,6211 |
| *P. aeruginosa* | 47 | 1,4963 | ,8348 |
| *E. fecilas* | 24 | 1,1768 | ,7467 |
| *E. faecium* | 51 | 1,4096 | ,7781 |
| *S. aureus* | 30 | ,7917 | ,6322 |

The below Table 5 shows the results for the patients said to be non-infected or infected separately and comparatively. It can be seen that the opsonisation levels for the non- infected samples are lower than those that have infected, as would be expected.

**Table 5: Comparison of opsonisation values in groups with and without culture growth.**

| | **Culture Negative** | | | **Culture Positive** | | |
|---|---|---|---|---|---|---|
| | **Number** | **AVG** | **SD** | **Number** | **AVG** | **SD** |
| *A. baumannii* | 42 | ,7465 | ,3800 | 21 | 1,7010 | ,5627 |
| *K. pneumoniae* | 24 | ,6398 | ,2467 | 12 | 1,2356 | ,6217 |
| *E. coli* | 18 | ,4523 | ,2728 | 29 | ,9729 | ,6935 |
| *P. aeruginosa* | 19 | ,9516 | ,5186 | 28 | 1,8658 | ,8110 |
| *E. fecilas* | 11 | ,5064 | ,1254 | 13 | 1,7441 | ,5429 |
| *E. faecium* | 21 | ,6250 | ,2316 | 30 | 1,9589 | ,4989 |
| *S. aureus* | 21 | ,4535 | ,1766 | 9 | 1,5810 | ,6081 |

It can be clearly seen from the Tables 4 and 5 that each bacteria has a different average opsonisation value, so determining the threshold for each bacteria is important to be accurate in determining the presence of the bacteria. ROC analysis was used to determine a cut-off threshold value for each strain of bacteria which results in the highest percentage for correctly classified patients when compared to the bacterial growth cell cultures.

**Table 6: ROC analysis results to determine appropriate cutting points for each bacterial strain.**

| | | | | Asymptotic 95% Confidence Interval | |
|---|---|---|---|---|---|
| | **Cut Off** | **Area** | **Std. Error** | **Lower Bound** | **Upper Bound** |
| *A. baumannii* | 1,1 | ,929 | ,032 | ,865 | ,992 |
| *K. pneumoniae* | 0,86 | ,854 | ,074 | ,709 | ,999 |
| *E. coli* | 0,82 | ,712 | ,076 | ,562 | ,861 |
| *P. aeruginosa* | 1,5 | ,825 | ,059 | ,710 | ,941 |
| *E. fecilas* | 0,85 | ,1,000 | ,000 | ,1,000 | ,1,000 |
| *E. faecium* | 0,91 | ,997 | ,004 | ,988 | ,1,005 |
| *S. aureus* | 0,72 | ,995 | ,009 | ,978 | ,1,012 |
| **Total** | 0,9 | ,871 | ,023 | ,826 | ,916 |

Opsonisation values above the cut off point indicate an infection in the patient who provided the sample and opsonisation values below the cut off point indicate that the patient is not infected.

### Example 3

Experimental data has been determined to show that the biological process and the system comprising the electode works and indeed that a status of the patient can be determined using the electrode. The results are shown in a table for a number of electrodes which have been functionalised with some of the bacteria and tested using samples from both infected patients and non-infected patients. The voltage settings for each of the bacteria was specifically determined and is unique to each bacterial strain.

**Table 7: Results showing measured response of electrode to different bacteria and different patient samples.**

| **BACTERIA** | **TYPE** | **VOLTAGE (V)** | **SENSOR READING (µA)** |
|---|---|---|---|
| *Acinetobacter baumannii* | Non-infected Patient | -0.55 | 196 |
| | Infected Patient | | 228 |
| *Staphylococcus aureus* | Non-infected Patient | -0.75 | 220 |
| | Infected Patient | | 263 |
| *Acinetobacter baumannii* | Non-infected Patient | -0.55 | 142 |
| | Infected Patient | | 233 |
| *Klebsiella pneumoniae* | Non-infected Patient | -0.1 | 241 |
| | Infected Patient | | 392 |
| *Acinetobacter baumannii* | Non-infected Patient | -0.55 | 45 |
| | Infected Patient | | 77 |
| *Pseudomonas aeruginosa* | Non-infected Patient | -0.775 | 265 |
| | Infected Patient | | 356 |

The results show that infected and non-infected patients can be differentiated by the measured output under electrochemical testing, where lower readings were measured by samples that were from non-infected patients compared to those from infected patients.

It will be appreciated from the above description that many featuresof the different examples are interchangeable and combinable. The disclosure extends to further examples comprising features from different examples combined together in ways not specifically mentioned. Indeed, there are many features presented in the above examples and it will be apparent to the skilled person that these may be advantageously combined with one another.

### Data

Data used in Example 1 concerning the opsonic activity for *Acinetobacter baumannii* of patients from: group 1 comprising 50 patients infected with *Acinetobacter baumannii*; group 2 comprising a positive control group of 30 patients; group 3 comprising 12 patients infected by another pathogen; group 4 comprising 30 non-infected hospital patients; and group 5 comprising 30 healthy people was 37.2% is shown below.

**Table 8: Opsonic activity of patients infected with Acinetobacter baumannii of group 1.**

| **AGE** | **SEX** | **CULTURED SAMPLE** | **IDENTIFIED BACTERIA** | **CFU (Colony-forming unit)** | **OPSONIC ACTIVTY FOR ACINETOBACTER BAUMANNII (%)** |
|---|---|---|---|---|---|
| 38 | 1 | ETA | *Acinetobacter baumannii* | 750,000 | 88 |
| 64 | 1 | ETA | *Acinetobacter baumannii* | 20,000 | 80 |
| 40 | 1 | BAL | *Acinetobacter baumannii* | 1,000,000 | 83 |
| 50 | 1 | ETA | *Acinetobacter baumannii* | 100,000 | 82 |
| 56 | 1 | ETA, TISSUE, HK | *Acinetobacter baumannii* | 1,000,000 | 100 |
| 82 | 1 | ETA | *Acinetobacter baumannii* | 500,000 | 100 |
| 62 | 0 | ETA | *Acinetobacter baumannii* | 1,000,000 | 79 |
| 42 | 0 | ETA | *Acinetobacter baumannii* | 1,000,000 | 87 |
| 75 | 1 | ETA | *Acinetobacter baumannii* | 1,000,000 | 98 |
| 74 | 0 | ETA | *Acinetobacter baumannii* | 100,000 | 100 |
| 90 | 1 | ETA | *Acinetobacter baumannii* | 1,000,000 | 100 |
| 50 | 1 | ETA | *Acinetobacter baumannii* | 1,000,000 | 89 |
| 65 | 1 | ETA | *Acinetobacter baumannii* | 1,000,000 | 98 |
| 62 | 1 | ETA | *Acinetobacter baumannii* | 1,000,000 | 90 |
| 44 | 0 | ETA | *Acinetobacter baumannii* | 1,000,000 | 94 |
| 42 | 1 | BAL | *Acinetobacter baumannii* | 1,000,000 | 97 |
| 37 | 1 | ETA | *Acinetobacter baumannii* | 1,000,000 | 96 |
| 75 | 1 | ETA, HK | *Acinetobacter baumannii* | 50,000 | 99 |
| 38 | 1 | BAL, HK | *Acinetobacter baumannii* | 1,000,000 | 96 |
| 82 | 0 | ETA, HK | *Acinetobacter baumannii* | 100,000 | 90 |
| 26 | 0 | ETA, HK | *Acinetobacter baumannii* | 1,000,000 | 100 |
| 17 | 1 | ETA, HK | *Acinetobacter baumannii* | 100,000 | 100 |
| 47 | 1 | ETA | *Acinetobacter baumannii* | 800,000 | 98 |
| 90 | 0 | ETA, DRAIN FLUID | *Acinetobacter baumannii* | 1,000,000 | 100 |
| 65 | 1 | ETA,HK | *Acinetobacter baumannii* | 1,000,000 | 100 |
| 76 | 1 | ETA,HK | *Acinetobacter baumannii* | 1,000,000 | 100 |
| 31 | 1 | ETA | *Acinetobacter baumannii* | 1,000,000 | 100 |
| 23 | 1 | ETA | *Acinetobacter baumannii* | 1,000,000 | 100 |
| 89 | 1 | ETA | *Acinetobacter baumannii* | 1,000,000 | 100 |
| 63 | 0 | ETA,HK | *Acinetobacter baumannii* | 1,000,000 | 100 |
| 66 | 1 | BAL | *Acinetobacter baumannii* | 100,000 | 100 |
| 37 | 1 | BAL | *Acinetobacter baumannii* | 1,000,000 | 98 |
| 46 | 1 | ETA | *Acinetobacter baumannii* | 1,000,000 | 100 |
| 61 | 1 | ETA | *Acinetobacter baumannii* | 1,000,000 | 85 |
| 40 | 0 | ETA | *Acinetobacter baumannii* | 600,00 | 46 |
| 51 | 0 | ETA | *Acinetobacter baumannii* | 500,000 | 100 |
| 68 | 1 | ETA | *Acinetobacter baumannii* | 200,000 | 100 |
| 77 | 0 | ETA | *Acinetobacter baumannii* | 1,000,000 | 100 |
| 62 | 1 | ETA | *Acinetobacter baumannii* | 1,000,000 | 66 |
| 86 | 1 | ETA | *Acinetobacter baumannii* | 1,000,000 | 70 |
| 69 | 0 | ETA | *Acinetobacter baumannii* | 1,000,000 | 100 |
| 14 | 1 | ETA | *Acinetobacter baumannii* | 1,000,000 | 100 |
| 29 | 1 | ETA | *Acinetobacter baumannii* | 1,000,000 | 86 |
| 34 | 0 | ETA | *Acinetobacter baumannii* | 1,000,000 | 100 |
| 81 | 0 | ETA | *Acinetobacter baumannii* | 1,000,000 | 100 |
| 35 | 1 | BAL | *Acinetobacter baumannii* | 500,00 | 100 |
| 67 | 0 | ETA, DRAIN FLUID | *Acinetobacter baumannii* | 100,000 | 31 |
| 53 | 0 | ETA | *Acinetobacter baumannii* | 100,000 | 97 |
| 79 | 0 | ETA | *Acinetobacter baumannii* | 1,000,000 | 98 |
| 46 | 1 | ETA, CSF | *Acinetobacter baumannii* | 1,000,000 | 95 |
| | | | | | **Average: 92.32** |

**Table 9: Opsonic activity of positive control group 2.**

| **AGE** | **SEX** | **CULTURED SAMPLE** | **IDENTIFIED BACTERIA** | **OPSONIC ACTIVITY FOR ACINETOBACTER BAUMANNII (%)** |
|---|---|---|---|---|
| 55 | 1 | Hemoculture | *Acinetobacter baumannii* | 99 |
| 48 | 1 | Hemoculture | *Acinetobacter baumannii* | 100 |
| 58 | 1 | Hemoculture | *Acinetobacter baumannii* | 100 |
| 76 | 0 | Hemoculture | *Acinetobacter baumannii* | 97 |
| 27 | 0 | Hemoculture | *Acinetobacter baumannii* | 98 |
| 77 | 1 | Hemoculture | *Acinetobacter baumannii* | 100 |
| 69 | 0 | Hemoculture | *Acinetobacter baumannii* | 96 |
| 82 | 0 | Hemoculture | *Acinetobacter baumannii* | 94 |
| 85 | 1 | Hemoculture | *Acinetobacter baumannii* | 96 |
| 85 | 1 | Hemoculture | *Acinetobacter baumannii* | 92 |
| 89 | 0 | Hemoculture | *Acinetobacter baumannii* | 100 |
| 73 | 1 | Hemoculture | *Acinetobacter baumannii* | 100 |
| 62 | 0 | Hemoculture | *Acinetobacter baumannii* | 100 |
| 81 | 1 | Hemoculture | *Acinetobacter baumannii* | 100 |
| 71 | 1 | Hemoculture | *Acinetobacter baumannii* | 96 |
| 66 | 0 | Hemoculture | *Acinetobacter baumannii* | 100 |
| 50 | 0 | Hemoculture | *Acinetobacter baumannii* | 96 |
| 60 | 0 | Hemoculture | *Acinetobacter baumannii* | 100 |
| 18 | 1 | Hemoculture | *Acinetobacter baumannii* | 92 |
| 18 | 1 | Hemoculture | *Acinetobacter baumannii* | 94 |
| 53 | 0 | Hemoculture | *Acinetobacter baumannii* | 86 |
| 35 | 1 | Hemoculture | *Acinetobacter baumannii* | 100 |
| 60 | 0 | Hemoculture | *Acinetobacter baumannii* | 100 |
| 64 | 1 | Hemoculture | *Acinetobacter baumannii* | 100 |
| 65 | 1 | Hemoculture | *Acinetobacter baumannii* | 100 |
| 39 | 1 | Hemoculture | *Acinetobacter baumannii* | 100 |
| 66 | 1 | Hemoculture | *Acinetobacter baumannii* | 100 |
| 59 | 1 | Hemoculture | *Acinetobacter baumannii* | 100 |
| 78 | 1 | Hemoculture | *Acinetobacter baumannii* | 100 |
| 46 | 1 | Hemoculture | *Acinetobacter baumannii* | 100 |
| | | | | **Average: 97.87** |

**Table 10: Group 3 data for patients infected with another pathogen.**

| | | | | **OPSONIC ACTIVITY (%)** | |
|---|---|---|---|---|---|
| **AGE** | **SEX** | **CULTURED SAMPLE** | **IDENTIFIED BACTERIA** | **FOR ISOLATED BACTERIA FROM SAMPLE** | **FOR ACINETOBACTER BAUMANNII** |
| 30 | 1 | Hemoculture | Vancomycin resistant | 100 | 17 |
| | | | Enterococcus spp. | | |
| 69 | 0 | Hemoculture | Escherichia coli | 100 | 22 |
| 60 | 1 | Hemoculture | Coagulase-negative staphylococci | 100 | 44 |
| 49 | 1 | Hemoculture | Extended-spectrum beta-lactamase-positive | 100 | 39 |
| | | | Escherichia coli | | |
| 37 | 0 | Hemoculture | Candida albicans | 100 | 31 |
| 72 | 0 | Hemoculture | Pseudomonas aeruginosa + Enterococcus sp. | 100 | 19 |
| 84 | 1 | Hemoculture | Candida albicans | 100 | 46 |
| 85 | 0 | Hemoculture | Coagulase-negative staphylococci | 100 | 50 |
| 83 | 1 | Hemoculture | Klebsiella pneumonia | 100 | 35 |
| 53 | 0 | Hemoculture | Methicillin-Resistant Staphylococcus epidermidis | 100 | 53 |
| 31 | 1 | Hemoculture | Streptococcus spp. | 100 | 54 |
| 56 | 0 | Hemoculture | Klebsiella sp. | 100 | 51 |
| | | | | | **Average: 38.42** |

**Table 11: Data for group 4, non-infected patients.**

| | | | | **OPSONIC ACTIVITY FOR ACINETOBACTER BAUMANNII (%)** |
|---|---|---|---|---|
| **AGE** | **SEX** | **CULTURED SAMPLE** | **IDENTIFIED BACTERIA** | |
| 35 | 1 | NONE | NONE | 46 |
| 44 | 0 | NONE | NONE | 66 |
| 44 | 1 | NONE | NONE | 44 |
| 56 | 1 | NONE | NONE | 17 |
| 64 | 1 | NONE | NONE | 3 |
| 40 | 0 | NONE | NONE | 4 |
| 54 | 0 | NONE | NONE | 9 |
| 78 | 0 | NONE | NONE | 2 |
| 68 | 1 | NONE | NONE | 84 |
| 74 | 1 | NONE | NONE | 75 |
| 88 | 1 | NONE | NONE | 86 |
| 59 | 1 | NONE | NONE | 81 |
| 21 | 0 | NONE | NONE | 21 |
| 81 | 1 | NONE | NONE | 12 |
| 84 | 1 | NONE | NONE | 75 |
| 47 | 0 | NONE | NONE | 70 |
| 71 | 0 | NONE | NONE | 76 |
| 78 | 0 | NONE | NONE | 78 |
| 50 | 1 | NONE | NONE | 85 |
| 48 | 0 | NONE | NONE | 94 |
| 65 | 0 | NONE | NONE | 87 |
| 78 | 1 | NONE | NONE | 74 |
| 40 | 1 | NONE | NONE | 32 |
| 91 | 1 | NONE | NONE | 17 |
| 70 | 1 | NONE | NONE | 6 |
| 57 | 0 | NONE | NONE | 10 |
| 53 | 1 | NONE | NONE | 20 |
| 55 | 0 | NONE | NONE | 20 |
| 59 | 0 | NONE | NONE | 37 |
| 58 | 0 | NONE | NONE | 44 |
| | | | | **Average: 45.83** |

**Table 12: Data for group 5, healthy people.**

| | | **OPSONIC ACTIVITY FOR ACINETOBACTER BAUMANNII (%)** |
|---|---|---|
| **AGE** | **SEX** | |
| 55 | 0 | 30 |
| 57 | 1 | 36 |
| 23 | 1 | 47 |
| 31 | 0 | 28 |
| 29 | 0 | 34 |
| 56 | 1 | 19 |
| 54 | 0 | 35 |
| 46 | 1 | 52 |
| 45 | 0 | 55 |
| 49 | 1 | 66 |
| 48 | 1 | 60 |
| 60 | 0 | 44 |
| 44 | 0 | 53 |
| 47 | 0 | 56 |
| 42 | 1 | 16 |
| 54 | 0 | 30 |
| 48 | 0 | 37 |
| 49 | 0 | 63 |
| 52 | 1 | 51 |
| 40 | 0 | 58 |
| 45 | 0 | 55 |
| 40 | 1 | 54 |
| 46 | 1 | 18 |
| 57 | 1 | 10 |
| 41 | 0 | 18 |
| 56 | 1 | 13 |
| 53 | 1 | 31 |
| 55 | 1 | 29 |
| 50 | 1 | 12 |
| 66 | 1 | 6 |
| | | **37.2** |

Example 2 provides a number of tables showing average opsonisation values for various bacterial strains. The below data is the raw data used in creating these results. In Table 13, group 1 represents *A*. *baumannii,* group 2 represents *K*. *pneumoniae,* group 3 represent *E*. *coli,* group 4 represents *P*. *aeruginosa,*group 5 represents *E*. *fecilas,* group 6 represents *E*. *faecium* and group 7 represents *S*. *aureus.*

**Table 13: Data used in determining the data presented in Tables 3, 4 and 5 as described in Example 2.**

| **GROUP 1** | **Sex** | **Bacterial Culture** | **Sample Type** | **WBC** | **Opsonisation** |
|---|---|---|---|---|---|
| 1 | 1 | 1 | 1 | 2 | **1,358** |
| 2 | 0 | 1 | 1 | 1 | **1,633** |
| 3 | 1 | 1 | 1 | 0 | **2,125** |
| 4 | 1 | 1 | 1 | 0 | **1,231** |
| 5 | 1 | 1 | 1 | 0 | **1,257** |
| 6 | 1 | 1 | 1 | 0 | **1,445** |
| 7 | 1 | 1 | 1 | 2 | **1,704** |
| 8 | 1 | 1 | 1 | 2 | **3,023** |
| 9 | 0 | 1 | 1 | 2 | **1,585** |
| 10 | 1 | 1 | 1 | 2 | **3,086** |
| 11 | 0 | 1 | 1 | 2 | **1,48** |
| 12 | 1 | 1 | 1 | 2 | **1,147** |
| 13 | 0 | 1 | 1 | 2 | **1,266** |
| 14 | 0 | 1 | 1 | 0 | **1.572** |
| 15 | 1 | 1 | 1 | 0 | **2,301** |
| 16 | 0 | 1 | 2 | 1 | **1,252** |
| 17 | 1 | 1 | 2 | 2 | **1,891** |
| 18 | 0 | 1 | 2 | 0 | **1,322** |
| 19 | 1 | 1 | 2 | 2 | **1,508** |
| 20 | 1 | 1 | 2 | 2 | **1,259** |
| 21 | 1 | 1 | 2 | 0 | **2,279** |
| 22 | 1 | 0 | 0 | 2 | **0,486** |
| 23 | 1 | 0 | 0 | 2 | **0,313** |
| 24 | 0 | 0 | 0 | 2 | **0,507** |
| 25 | 0 | 0 | 0 | 2 | **0,601** |
| 26 | 1 | 0 | 0 | 0 | **0,616** |
| 27 | 1 | 0 | 0 | 2 | **0,74** |
| 28 | 1 | 0 | 0 | 2 | **0,587** |
| 29 | β | 0 | 0 | 0 | **0,306** |
| 30 | 0 | 0 | 0 | 2 | **0,57** |
| 31 | 1 | 0 | 0 | 0 | **0,656** |
| 32 | 1 | 0 | 0 | 0 | **0,602** |
| 33 | 0 | 0 | 2 | 0 | **0,486** |
| 34 | 0 | 0 | 2 | 2 | **1,035** |
| 35 | 0 | 0 | 2 | 2 | **0,798** |
| 36 | 0 | 0 | 0 | 0 | **0,955** |
| 37 | 1 | 0 | 2 | 2 | **1,149** |
| 38 | 0 | 0 | 0 | 0 | **0,665** |
| 39 | 0 | 0 | 2 | 2 | **0,667** |
| 40 | 0 | 0 | 1 | 2 | **0,896** |
| 41 | 1 | 0 | 0 | 2 | **0,7** |
| 42 | 0 | 0 | 0 | 0 | **0.373** |
| 43 | 0 | 0 | 0 | 2 | **0,499** |
| 44 | 1 | 0 | 2 | 2 | **0,634** |
| 45 | 1 | 0 | 0 | 2 | **0,602** |
| 46 | 0 | 0 | 2 | 2 | **0,631** |
| 47 | 1 | 0 | 2 | 1 | **0,683** |
| 48 | 1 | 0 | 2 | 0 | **0,662** |
| 49 | 1 | 0 | 2 | 1 | **0,311** |
| 50 | 0 | 0 | 2 | 2 | **0,48** |
| 51 | 0 | 0 | 2 | 2 | **0,53** |
| 52 | 0 | 0 | 2 | 2 | **1,577** |
| 53 | 0 | 0 | 2 | 2 | **1,641** |
| 54 | 0 | 0 | 2 | 2 | **0,846** |
| 55 | 1 | 0 | 2 | 2 | **1,643** |
| 56 | 0 | 0 | 2 | 1 | **1,011** |
| 57 | 0 | 0 | 2 | 1 | **1,688** |
| 58 | 0 | 0 | 2 | 1 | **1,501** |
| 59 | 0 | 0 | 2 | 1 | **1,208** |
| 60 | 1 | 0 | 0 | 2 | **0,383** |
| 61 | 0 | 0 | 0 | 2 | **0,367** |
| 62 | 1 | 0 | 0 | 0 | **0,518** |
| 63 | 0 | 0 | 2 | 2 | **0,453** |

| **GROUP 2** | **Sex** | **Bacterial Culture** | **Sample Type** | **WBC** | **Opsonisation** |
|---|---|---|---|---|---|
| 1 | 1 | 1 | 1 | 0 1 | **0,99** |
| 2 | 1 | 1 | 1 | 2 | **0,395** |
| 3 | 1 | 1 | 1 | 2 | **1,21** |
| 4 | 0 | 1 | 1 | 0 | **2,814** |
| 5 | 1 | 1 | 2 | 1 | **0,66** |
| 6 | 1 | 1 | 2 | 0 | **1,126** |
| 7 | 0 | 1 | 2 | 1 | **1,402** |
| 8 | 1 | 1 | 2 | 2 | **0,909** |
| 9 | 1 | 1 | 2 | 2 | **1,28** |
| 10 | 0 | 1 | 2 | 1 | **0,887** |
| 11 | 1 | 1 | 2 | 2 | **1,868** |
| 12 | 0 | 1 | 2 | 2 | **1.296** |
| 13 | 0 | 0 | 0 | 2 | **0,417** |
| 14 | 0 | 0 | 2 | 2 | **0,378** |
| 15 | 1 | 0 | 2 | 2 | **0,511** |
| 16 | 0 | 0 | 2 | 2 | **0,586** |
| 17 | 0 | 0 | 2 | 2 | **0,464** |
| 18 | 0 | 0 | 2 | 2 | **0,938** |
| 19 | 0 | 0 | 2 | 2 | **0,604** |
| 20 | 1 | 0 | 2 | 2 | **0,556** |
| 21 | 0 | 0 | 2 | 1 | **1,012** |
| 22 | 0 | 0 | 2 | 1 | **1,164** |
| 23 | 0 | 0 | 2 | 2 | **0,702** |
| 24 | 0 | 0 | 2 | 2 | **0,326** |
| 25 | 0 | 0 | 0 | 2 | **0,976** |
| 26 | 0 | 0 | 0 | 2 | **1,155** |
| 27 | 1 | 0 | 0 | 2 | **0,621** |
| 28 | 1 | 0 | 0 | 2 | **0,548** |
| 29 | 0 | 0 | 0 | 2 | **0,658** |
| 30 | 1 | 0 | 2 | 2 | **0,831** |
| 31 | 1 | 0 | 0 | 2 | **0,585** |
| 32 | 1 | 0 | 0 | 0 | **0,394** |
| 33 | 0 | 0 | 0 | 0 | **0,372** |
| 34 | 0 | 0 | 0 | 0 | **0,515** |
| 35 | 1 | 0 | 0 | 2 | **0,433** |
| 36 | 0 | 0 | 0 | 0 | **0,61** |

| **GROUP 3** | **Sex** | **Bacterial Culture** | **Sample Type** | **WBC** | **Opsonisation** |
|---|---|---|---|---|---|
| 1 | 0 | 1 | 1 | 0 | **0,465** |
| 2 | 0 | 1 | 1 | 2 | **0,155** |
| 3 | 1 | 1 | 1 | 2 | **0,326** |
| 4 | 1 | 1 | 1 | 2 | **0,619** |
| 5 | 1 | 1 | 1 | 2 | **1,07** |
| 6 | 1 | 1 | 1 | 0 | **1,294** |
| 7 | 1 | 1 | 1 | 2 | **0,25** |
| 8 | 1 | 1 | 1 | 2 | **1,609** |
| 9 | 0 | 1 | 1 | 2 | **0,201** |
| 10 | 1 | 1 | 1 | 0 | **1,06** |
| 11 | 0 | 1 | 1 | 2 | **2,138** |
| 12 | 0 | 1 | 1 | 0 | **1,005** |
| 13 | 1 | 1 | 1 | 2 | **0,763** |
| 14 | 1 | 1 | 2 | 0 | **0,323** |
| 15 | 1 | 1 | 2 | 1 | **0,337** |
| 16 | 1 | 1 | 2 | 0 | **0,299** |
| 17 | 0 | 1 | 2 | 2 | **0,166** |
| 18 | 1 | 1 | 2 | 2 | **0,218** |
| 19 | 0 | 1 | 2 | 2 | **1,425** |
| 20 | 0 | 1 | 2 | 2 | **2,308** |
| 21 | 0 | 1 | 2 | 1 | **1,139** |
| 22 | 1 | 1 | 2 | 2 | **2,378** |
| 23 | 1 | 1 | 2 | 2 | **1,075** |
| 24 | 0 | 1 | 2 | 2 | **1,385** |
| 25 | 1 | 1 | 1 | 2 | **0,263** |
| 26 | 1 | 1 | 2 | 2 | **1,5** |
| 27 | 1 | 1 | 2 | 2 | **2,012** |
| 28 | 0 | 1 | 2 | 2 | **1,603** |
| 29 | 0 | 1 | 2 | 0 | **0,828** |
| 30 | 1 | 0 | 0 | 0 | **0,793** |
| 31 | 0 | 0 | 0 | 2 | **0,613** |
| 32 | 0 | 0 | 2 | 2 | **0,068** |
| 33 | 1 | 0 | 2 | 1 | **0,380** |
| 34 | 1 | 0 | 2 | 0 | **0,490** |
| 35 | 1 | 0 | 2 | 0 | **0,071** |
| 36 | 0 | 0 | 2 | 2 | **0,063** |
| 37 | 0 | 0 | 0 | 2 | **0,077** |
| 38 | 1 | 0 | 0 | 0 | **0,416** |
| 39 | 0 | 0 | 0 | 1 | **0,653** |
| 40 | 1 | 0 | 0 | 0 | **0,477** |
| 41 | 1 | 0 | 0 | 0 | **0,337** |
| 42 | 0 | 0 | 0 | 2 | **0,163** |
| 43 | 0 | 0 | 0 | 2 | **0,553** |
| 44 | 1 | 0 | 2 | 2 | **0,846** |
| 45 | 1 | 0 | 0 | 2 | **0,595** |
| 46 | 0 | 0 | 0 | 0 | **0,783** |
| 47 | 1 | 0 | 0 | 2 | **0,764** |

| **GROUP 4** | **Sex** | **Bacterial Culture** | **Sample Type** | **WBC** | **Opsonisation** |
|---|---|---|---|---|---|
| 1 | 1 | 1 | 1 | 0 | **2,447** |
| 2 | 1 | 1 | 1 | 1 | **2,679** |
| 3 | 0 | 1 | 2 | 1 | **0,787** |
| 4 | 1 | 1 | 1 | 2 | **1,264** |
| 5 | 1 | 1 | 1 | 2 | **2,229** |
| 6 | 1 | 1 | 1 | 1 | **1,476** |
| 7 | 1 | 1 | 1 | 2 | **1,562** |
| 8 | 1 | 1 | 1 | 0 | **2,978** |
| 9 | 0 | 1 | 1 | 0 | **1,117** |
| 10 | 1 | 1 | 1 | 1 | **0,922** |
| 11 | 1 | 1 | 1 | 0 | **3,423** |
| 12 | 1 | 1 | 1 | 2 | **2,49** |
| 13 | 0 | 1 | 1 | 0 | **2,68** |
| 14 | 0 | 1 | 1 | 0 | **1,173** |
| 15 | 1 | 1 | 1 | 2 | **0,862** |
| 16 | 0 | 1 | 1 | 2 | **0,532** |
| 17 | 1 | 1 | 1 | 2 | **1,595** |
| 18 | 0 | 1 | 2 | 1 | **2,631** |
| 19 | 1 | 1 | 2 | 0 | **2,705** |
| 20 | 1 | 1 | 2 | 0 | **1,976** |
| 21 | 0 | 1 | 2 | 1 | **1,581** |
| 22 | 1 | 1 | 2 | 0 | **2,071** |
| 23 | 1 | 1 | 2 | 2 | **1,278** |
| 24 | 1 | 1 | 2 | 0 | **1,656** |
| 25 | 1 | 1 | 2 | 2 | **2,078** |
| 26 | 1 | 1 | 2 | 2 | **3,286** |
| 27 | 0 | 1 | 2 | 0 | **0,673** |
| 28 | 1 | 1 | 2 | 0 | **2,094** |
| 29 | 1 | 0 | 0 | 2 | **0,441** |
| 30 | 1 | 0 | 0 | 2 | **0,609** |
| 31 | 0 | 0 | 0 | 2 | **0,749** |
| 32 | 1 | 0 | 2 | 2 | **0,653** |
| 33 | 0 | 0 | 0 | 0 | **0,207** |
| 34 | 0 | 0 | 2 | 2 | **0,866** |
| 35 | 0 | 0 | 0 | 0 | **0,855** |
| 36 | 1 | 0 | 2 | 2 | **0,855** |
| 37 | 1 | 0 | 2 | 2 | **0,788** |
| 38 | 0 | 0 | 0 | 2 | **1,457** |
| 39 | 0 | 0 | 2 | 2 | **1,213** |
| 40 | 1 | 0 | 2 | 1 | **1,408** |
| 41 | 1 | 0 | 2 | 0 | **1,292** |
| 42 | 0 | 0 | 2 | 2 | **0,986** |
| 43 | 0 | 0 | 2 | 2 | **1,442** |
| 44 | 0 | 0 | 2 | 2 | **1,924** |
| 45 | 0 | 0 | 2 | 2 | **1,854** |
| 46 | 0 | 0 | 2 | 1 | **0,06** |
| 47 | 0 | 0 | 0 | 0 | **0,422** |

| **GROUP 5** | **Sex** | **Bacterial Culture** | **Sample Type** | **WBC** | **Opsonisation** |
|---|---|---|---|---|---|
| 1 | 0 | 1 | 1 | 0 | **2,048** |
| 2 | 1 | 1 | 1 | 0 | **2,578** |
| 3 | 0 | 1 | 1 | 2 | **2,424** |
| 4 | 1 | 1 | 1 | 0 | **2,381** |
| 5 | 0 | 1 | 1 | 2 | **1,073** |
| 6 | 1 | 1 | 1 | 0 | **1,014** |
| 7 | 0 | 1 | 2 | 2 | **1,306** |
| 8 | 1 | 1 | 2 | 0 | **2,029** |
| 9 | 0 | 1 | 2 | 2 | **1,31** |
| 10 | 1 | 1 | 2 | 0 | **1,307** |
| 11 | 0 | 1 | 2 | 2 | **2,129** |
| 12 | 1 | 1 | 2 | 0 | **1,46** |
| 13 | 1 | 1 | 2 | 2 | **1,614** |
| 14 | 0 | 0 | 2 | 2 | **0,679** |
| 15 | 0 | 0 | 0 | 0 | **0,522** |
| 16 | 1 | 0 | 0 | 2 | **0,644** |
| 17 | 1 | 0 | 2 | 2 | **0,481** |
| 18 | 1 | 0 | 0 | 2 | **0,456** |
| 19 | 1 | 0 | 0 | 2 | **0,668** |
| 20 | 1 | 0 | 0 | 0 | **0,58** |
| 21 | 0 | 0 | 0 | 0 | **0,661** |
| 22 | 0 | 0 | 0 | 0 | **0,292** |
| 23 | 1 | 0 | 0 | 2 | **0,498** |
| 24 | 0 | 0 | 0 | 0 | **0,29** |

| **GROUP 6** | **Sex** | **Bacterial Culture** | **Sample Type** | **WBC** | **Opsonisation** |
|---|---|---|---|---|---|
| 1 | 1 | 1 | 1 | 0 | **1,959** |
| 2 | 1 | 1 | 1 | 0 | **1,607** |
| 3 | 1 | 1 | 1 | 2 | **1,176** |
| 4 | 1 | 1 | 1 | 0 | **2,438** |
| 5 | 1 | 1 | 1 | 2 | **2,136** |
| 6 | 0 | 1 | 1 | 2 | **1,6** |
| 7 | 0 | 1 | 1 | 2 | **1,462** |
| 8 | 0 | 1 | 1 | 0 | **1,598** |
| 9 | 0 | 1 | 1 | 1 | **1,581** |
| 10 | 1 | 1 | 1 | 2 | **2,088** |
| 11 | 1 | 1 | 1 | 2 | **0,91** |
| 12 | 0 | 1 | 2 | 2 | **2,408** |
| 13 | 1 | 1 | 2 | 2 | **2,076** |
| 14 | 1 | 1 | 2 | 1 | **2,741** |
| 15 | 0 | 1 | 2 | 2 | **2,461** |
| 16 | 1 | 1 | 2 | 0 | **2,469** |
| 17 | 0 | 1 | 2 | 0 | **2,161** |
| 18 | 1 | 1 | 2 | 2 | **2,566** |
| 19 | 0 | 1 | 2 | 2 | **1,71** |
| 20 | 0 | 1 | 2 | 1 | **1,526** |
| 21 | 0 | 1 | 2 | 2 | **1,71** |
| 22 | 0 | 1 | 2 | 2 | **2,347** |
| 23 | 1 | 1 | 2 | 2 | **1,529** |
| 24 | 1 | 1 | 2 | 0 | **1,012** |
| 25 | 1 | 1 | 2 | 2 | **2,16** |
| 26 | 1 | 1 | 2 | 0 | **2,052** |
| 27 | 1 | 1 | 2 | 2 | **2,328** |
| 28 | 0 | 1 | 2 | 2 | **2,074** |
| 29 | 1 | 1 | 2 | 2 | **1,961** |
| 30 | 0 | 1 | 2 | 2 | **2,943** |
| 31 | 0 | 0 | 0 | 2 | **0,359** |
| 32 | 0 | 0 | 2 | 2 | **0,616** |
| 33 | 0 | 0 | 2 | 2 | **0,39** |
| 34 | 0 | 0 | 2 | 1 | **0,277** |
| 35 | 1 | 0 | 0 | 2 | **0,281** |
| 36 | 0 | 0 | 0 | 1 | **0,784** |
| 37 | 0 | 0 | 0 | 1 | **0,796** |
| 38 | 1 | 0 | 2 | 0 | **0.537** |
| 39 | 0 | 0 | 0 | 2 | **0,608** |
| 40 | 0 | 0 | 0 | 2 | **0,432** |
| 41 | 0 | 0 | 0 | 2 | **0,346** |
| 42 | 1 | 0 | 0 | 2 | **0,984** |
| 43 | 0 | 0 | 0 | 2 | **0,846** |
| 44 | 1 | 0 | 0 | 2 | **0,954** |
| 45 | 0 | 0 | 0 | 0 | **0,768** |
| 46 | 1 | 0 | 0 | 2 | **0,671** |
| 47 | 1 | 0 | 0 | 0 | **0,853** |
| 48 | 0 | 0 | 0 | 0 | **0,738** |
| 49 | 0 | 0 | 0 | 0 | **0,716** |
| 50 | 1 | 0 | 0 | 2 | **0,893** |
| 51 | 0 | 0 | 0 | 0 | **0,375** |

| **GROUP 7** | **Sex** | **Bacterial Culture** | **Sample Type** | **WBC** | **Opsonisation** |
|---|---|---|---|---|---|
| 1 | 1 | 1 | 2 | 0 | **1,963** |
| 2 | 0 | 1 | 1 | 2 | **1,804** |
| 3 | 1 | 1 | 1 | 1 | **2,409** |
| 4 | 1 | 1 | 1 | 2 | **1,739** |
| 5 | 0 | 1 | 1 | 2 | **2,180** |
| 6 | 0 | 1 | 1 | 2 | **1,641** |
| 7 | 1 | 1 | 1 | 0 | **0,728** |
| 8 | 1 | 1 | 1 | 0 | **0,948** |
| 9 | 1 | 1 | 1 | 2 | **0,827** |
| 10 | 0 | 0 | 0 | 0 | **0,659** |
| 11 | 1 | 0 | 2 | 1 | **0,271** |
| 12 | 0 | 0 | 2 | 2 | **0,344** |
| 13 | 0 | 0 | 2 | 1 | **0,697** |
| 14 | 1 | 0 | 0 | 0 | **0,545** |
| 15 | 0 | 0 | 0 | 2 | **0,523** |
| 16 | 1 | 0 | 0 | 0 | **0,469** |
| 17 | 0 | 0 | 2 | 2 | **0,668** |
| 18 | 0 | 0 | 2 | 2 | **0,826** |
| 19 | 1 | 0 | 0 | 2 | **0,644** |
| 20 | 1 | 0 | 0 | 2 | **0,571** |
| 21 | 0 | 0 | 0 | 2 | **0,273** |
| 22 | 1 | 0 | 2 | 2 | **0,372** |
| 23 | 1 | 0 | 0 | 2 | **0,360** |
| 24 | 0 | 0 | 0 | 0 | **0,389** |
| 25 | 1 | 0 | 0 | 2 | **0,328** |
| 26 | 1 | 0 | 0 | 0 | **0,341** |
| 27 | 0 | 0 | 0 | 0 | **0,527** |
| 28 | 0 | 0 | 0 | 0 | **0,227** |
| 29 | 1 | 0 | 0 | 2 | **0,297** |
| 30 | 0 | 0 | 0 | 0 | **0,193** |

## Claims

1. A system for detecting an infection with a bacterial pathogen strain, the system comprising:
an electrode functionalised with the surface proteins of the bacterial pathogen strain; and
a controller configured to communicate with the electrode to perform an electrochemical test of a biological sample from a subject, the biological sample deposited on the electrode, wherein the electrochemical test measures a binding energy of one or more biomarkers in the biological sample with the surface proteins of the bacterial pathogen strain functionalised on the electrode to determine whether the subject has an immune response to the bacterial pathogen strain indicative of an infection with the bacterial pathogen strain,
wherein the controller is an electronic device,
wherein the biomarkers comprise one or more of C3b, IgG1 and/or IgG3;
and wherein the system is configured to determine whether the subject has an infection with the bacteria based on a determination that the binding energy exceeds a threshold value.

2. The system of claim 1, wherein the surface proteins of the bacterial pathogen strain comprise complement binding proteins, C3b binding proteins, immunoglobulin binding proteins; CR1 binding proteins; FcR binding proteins; extracellular complement binding proteins; Protein A, Immunoglobulin-binding protein 1; membrane proteins; and outer membrane proteins.

3. The system of claim 1 or claim 2, wherein the biological sample is selected from the group consisting of: a blood sample; a urine sample; an endotracheal aspiration sample; a bronchoalveolar lavage sample; and other extracellular fluid sample of the person.

4. The system of any one of claims 1 to 3, wherein the surface proteins functionalised on the electrode are immobilized on a surface of the electrode

5. The system of claim 4, wherein the immobilized surface proteins comprise a coating on the surface of the electrode.

6. The system of any one of claims 3 to 5, wherein the electrode comprises a plurality of conductive elements and wherein the controller is configured to perform the electrochemical test by measuring the binding energy of the one or more biomarkers in the blood sample with the surface proteins functionalised on the electrode by applying a voltage differential across at least some of the plurality of conductive elements.

7. The system of any one of claims 1 to 6, wherein the controller is configured to select the threshold value based on the infection being detected.

8. The system of any one of claims 1 to 7, wherein the threshold value is between -3.4 V and 3.4 V.

9. A method of detecting an infection with a bacterial pathogenic strain, the method comprising:
functionalising an electrode with the surface proteins of the bacterial pathogenic strain; and
performing an electrochemical test of a biological sample from a subject, the biological sample deposited on the electrode,
wherein the electrochemical test comprises measuring a binding energy of one or more biomarkers in the biological sample with the surface proteins functionalised on the electrode to determine whether the subject has an immune response to the pathogenic strain indicative of an infection with the bacterial pathogenic strain;
wherein the biomarkers comprise one or more of C3b, IgG1 and/or IgG3;
and wherein the method comprises determining whether the subject has an infection with the bacteria based on a determination that the binding energy exceeds a threshold value.

10. The method of claim 9, wherein functionalising the electrode comprises immobilizing the surface proteins onto a surface of the electrode.

11. The method of claim 10, wherein functionalising the electrode comprises coating the surface of the electrode with the surface proteins.

12. The method of any one of claims 9 to 11, wherein the threshold value is selected based on the infection being detected.

13. The method of any one of claims 9 to 12, wherein the threshold value is between -3.4 V and 3.4 V.

14. The method of any one of claims 9 to 13 wherein the electrode comprises a plurality of conductive elements and wherein the method comprises performing an electrochemical test by measuring a binding energy of the one or more biomarkers in a blood sample with the surface proteins functionalised on the electrode by applying a voltage differential across at least some of the plurality of conductive elements.

## Patentansprüche

1. System zum Detektieren einer Infektion mit einem pathogenen Bakterienstamm, wobei das System umfasst:
eine Elektrode, die mit den Oberflächenproteinen des pathogenen Bakterienstamms funktionalisiert ist; und
eine Steuerung, die ausgestaltet ist, um mit der Elektrode zu kommunizieren, um einen elektrochemischen Test einer biologischen Probe von einem Subjekt durchzuführen, wobei die biologische Probe auf der Elektrode abgeschieden wird, wobei der elektrochemische Test eine Bindungsenergie von einem oder mehreren Biomarkern in der biologischen Probe mit den Oberflächenproteinen des pathogenen Bakterienstamms misst, der auf der Elektrode funktionalisiert ist, um zu bestimmen, ob das Subjekt eine Immunreaktion auf den pathogenen Bakterienstamm hat, die eine Infektion mit dem pathogenen Bakterienstamm angibt,
wobei die Steuerung eine elektronische Vorrichtung ist,
wobei die Biomarker ein oder mehrere von C3b, IgG1 und/oder IgG3 umfassen;
und wobei das System ausgestaltet ist, um basierend auf einer Bestimmung, dass die Bindungsenergie einen Schwellenwert überschreitet, zu bestimmen, ob das Subjekt eine Infektion mit dem Bakterium hat.

2. System nach Anspruch 1, wobei die Oberflächenproteine des pathogenen Bakterienstamms Komplementbindungsproteine, C3b-Bindungsproteine, Immunoglobulinbindungsproteine; CR1-Bindungsproteine; FcR-Bindungsproteine; extrazelluläre Komplementbindungsproteine; Protein A, Immunoglobulinbindungsprotein 1; Membranproteine und Außenmembranproteine umfasst.

3. System nach Anspruch 1 oder Anspruch 2, wobei die biologische Probe ausgewählt ist aus der Gruppe bestehend aus: einer Blutprobe; einer Urinprobe; einer endotrachealen Aspirationsprobe; einer bronchoalveolären Lavageprobe und anderer extrazellulärer Flüssigkeitsprobe der Person.

4. System nach einem der Ansprüche 1 bis 3, wobei die auf der Elektrode funktionalisierten Oberflächenproteine auf einer Oberfläche der Elektrode immobilisiert sind.

5. System nach Anspruch 4, wobei die immobilisierten Oberflächenproteine eine Beschichtung auf der Oberfläche der Elektrode umfassen.

6. System nach einem der Ansprüche 3 bis 5, wobei die Elektrode eine Vielzahl von leitfähigen Elementen umfasst, und wobei die Steuerung ausgestaltet ist, um den elektrochemischen Test durchzuführen, indem die Bindungsenergie des einen oder der mehreren Biomarker in der Blutprobe mit den auf der Elektrode funktionalisierten Oberflächenproteinen gemessen wird, indem eine Spannungsdifferenz über mindestens einige von der Vielzahl der leitfähigen Elemente angelegt wird.

7. System nach einem der Ansprüche 1 bis 6, wobei die Steuerung ausgestaltet ist, um den Schwellenwert basierend auf der Infektion auszuwählen, welche detektiert wird.

8. System nach einem der Ansprüche 1 bis 7, wobei der Schwellenwert zwischen -3,4 V und 3,4 V liegt.

9. Verfahren zum Detektieren einer Infektion mit einem pathogenen Bakterienstamm, wobei das Verfahren umfasst:
Funktionalisieren einer Elektrode mit den Oberflächenproteinen des pathogenen Bakterienstamms; und
Durchführen eines elektrochemischen Tests einer biologischen Probe von einem Subjekt, wobei die biologische Probe auf der Elektrode abgeschieden wird,
wobei der elektrochemische Test Messen einer Bindungsenergie von einem oder mehreren Biomarkern in der biologischen Probe mit den auf der Elektrode funktionalisierten Oberflächenproteinen umfasst, um zu bestimmen, ob das Subjekt eine Immunreaktion auf den pathogenen Stamm hat, die eine Infektion mit dem pathogenen Bakterienstamm angibt;
wobei die Biomarker ein oder mehrere von C3b, IgG1 und/oder IgG3 umfassen;
und wobei das Verfahren Bestimmen, ob das Subjekt eine Infektion mit dem Bakterium hat, basierend auf einer Bestimmung, dass die Bindungsenergie einen Schwellenwert überschreitet, umfasst.

10. Verfahren nach Anspruch 9, wobei Funktionalisieren der Elektrode Immobilisieren der Oberflächenproteine auf einer Oberfläche der Elektrode umfasst.

11. Verfahren nach Anspruch 10, wobei Funktionalisieren der Elektrode Beschichten der Oberfläche der Elektrode mit den Oberflächenproteinen umfasst.

12. Verfahren nach einem der Ansprüche 9 bis 11, wobei der Schwellenwert basierend auf der Infektion, welche detektiert wird, ausgewählt wird.

13. Verfahren nach einem der Ansprüche 9 bis 12, wobei der Schwellenwert zwischen -3,4 V und 3,4 V liegt.

14. Verfahren nach einem der Ansprüche 9 bis 13, wobei die Elektrode eine Vielzahl von leitfähigen Elementen umfasst, und wobei das Verfahren Durchführen eines elektrochemischen Tests durch Messen einer Bindungsenergie des einen oder der mehreren Biomarker in einer Blutprobe mit den auf der Elektrode funktionalisierten Oberflächenproteinen umfasst, indem eine Spannungsdifferenz über mindestens einige von der Vielzahl der leitfähigen Elemente angelegt wird.

## Revendications

1. Système pour détecter une infection par une souche bactérienne pathogène, le système comprenant :
une électrode fonctionnalisée avec les protéines de surface de la souche bactérienne pathogène ; et
un contrôleur configuré pour communiquer avec l'électrode afin de réaliser un test électrochimique sur un échantillon biologique d'un sujet, l'échantillon biologique étant déposé sur l'électrode, où le test électrochimique mesure une énergie de liaison d'un ou plusieurs biomarqueurs dans l'échantillon biologique avec les protéines de surface de la souche bactérienne pathogène fonctionnalisée sur l'électrode afin de déterminer si le sujet présente une réponse immunitaire à la souche bactérienne pathogène indiquant une infection par la souche bactérienne pathogène,
où le contrôleur est un dispositif électronique,
où les biomarqueurs comprennent un ou plusieurs parmi C3b, IgG1 et/ou IgG3 ;
et où le système est configuré pour déterminer si le sujet présente une infection par la bactérie sur la base d'une détermination que l'énergie de liaison dépasse une valeur seuil.

2. Système selon la revendication 1, où les protéines de surface de la souche bactérienne pathogène comprennent des protéines de liaison au complément, des protéines de liaison au C3b, des protéines de liaison aux immunoglobulines ; des protéines de liaison au CR1 ; des protéines de liaison au FcR ; des protéines extracellulaires de liaison au complément ; la protéine A, la protéine 1 de liaison aux immunoglobulines ; des protéines membranaires ; et des protéines de membrane externe.

3. Système selon la revendication 1 ou la revendication 2, où l'échantillon biologique est choisi dans le groupe constitué par : un échantillon de sang ; un échantillon d'urine ; un échantillon d'aspiration endotrachéale ; un échantillon de lavage broncho-alvéolaire ; et un autre échantillon de liquide extracellulaire de la personne.

4. Système selon l'une quelconque des revendications 1 à 3, où les protéines de surface fonctionnalisées sur l'électrode sont immobilisées sur une surface de l'électrode.

5. Système selon la revendication 4, où les protéines de surface immobilisées comprennent un revêtement sur la surface de l'électrode.

6. Système selon l'une quelconque des revendications 3 à 5, où l'électrode comprend une pluralité d'éléments conducteurs et où le contrôleur est configuré pour réaliser le test électrochimique en mesurant l'énergie de liaison des un ou plusieurs biomarqueurs dans l'échantillon de sang avec les protéines de surface fonctionnalisées sur l'électrode en appliquant un différentiel de tension sur au moins certains de la pluralité d'éléments conducteurs.

7. Système selon l'une quelconque des revendications 1 à 6, où le contrôleur est configuré pour sélectionner la valeur seuil sur la base de l'infection détectée.

8. Système selon l'une quelconque des revendications 1 à 7, où la valeur seuil est comprise entre -3,4 V et 3,4 V.

9. Procédé de détection d'une infection par une souche bactérienne pathogène, le procédé comprenant :
la fonctionnalisation d'une électrode avec les protéines de surface de la souche bactérienne pathogène ; et
la réalisation d'un test électrochimique sur un échantillon biologique d'un sujet, l'échantillon biologique étant déposé sur l'électrode,
où le test électrochimique comprend la mesure d'une énergie de liaison d'un ou plusieurs biomarqueurs dans l'échantillon biologique avec les protéines de surface fonctionnalisées sur l'électrode afin de déterminer si le sujet présente une réponse immunitaire à la souche pathogène indiquant une infection par la souche bactérienne pathogène ;
où les biomarqueurs comprennent un ou plusieurs parmi C3b, IgG1 et/ou IgG3 ;
et où le procédé comprend la détermination de si le sujet présente une infection par la bactérie sur la base d'une détermination que l'énergie de liaison dépasse une valeur seuil.

10. Procédé selon la revendication 9, où la fonctionnalisation de l'électrode comprend l'immobilisation des protéines de surface sur une surface de l'électrode.

11. Procédé selon la revendication 10, où la fonctionnalisation de l'électrode comprend le revêtement de la surface de l'électrode avec les protéines de surface.

12. Procédé selon l'une quelconque des revendications 9 à 11, où la valeur seuil est sélectionnée sur la base de l'infection détectée.

13. Procédé selon l'une quelconque des revendications 9 à 12, où la valeur seuil est comprise entre -3,4 V et 3,4 V.

14. Procédé selon l'une quelconque des revendications 9 à 13, où l'électrode comprend une pluralité d'éléments conducteurs et où le procédé comprend la réalisation d'un test électrochimique en mesurant une énergie de liaison des un ou plusieurs biomarqueurs dans un échantillon de sang avec les protéines de surface fonctionnalisées sur l'électrode en appliquant un différentiel de tension sur au moins certains de la pluralité d'éléments conducteurs.
